(19) Europäisches Patentamt
European Patent Office
Office européen des brevets

(11) Veröffentlichungsnummer: **0 178 450 B1**

(12) # EUROPÄISCHE PATENTSCHRIFT

(45) Veröffentlichungstag der Patentschrift: **17.04.91**

(51) Int. Cl.[5]: **G01N 33/533**, G01N 33/574, G01N 33/76, C07F 15/00

(21) Anmeldenummer: **85111377.9**

(22) Anmeldetag: **09.09.85**

(54) **Metallkomplexe, an die ein immunologisch aktives Material gekoppelt werden kann bzw. ist, deren Herstellung und Verwendung.**

(30) Priorität: **17.09.84 CH 4433/84**
**10.07.85 CH 2984/85**

(43) Veröffentlichungstag der Anmeldung:
**23.04.86 Patentblatt 86/17**

(45) Bekanntmachung des Hinweises auf die Patenterteilung:
**17.04.91 Patentblatt 91/16**

(84) Benannte Vertragsstaaten:
**AT BE CH DE FR GB IT LI NL SE**

(56) Entgegenhaltungen:
**US-A- 4 205 952**
**US-A- 4 283 382**
**US-A- 4 293 310**
**US-A- 4 341 757**
**US-A- 4 374 120**

**CHEMICAL ABSTRACTS, Band 98, Nr. 14, 04 April 1983, Columbus, OH (US); D.S.C.BLACK et al., S. 174, Nr. 118503t**

(73) Patentinhaber: **F. HOFFMANN-LA ROCHE AG**
**Postfach 3255**
**CH-4002 Basel(CH)**

(72) Erfinder: **Müller, Francis**
**Seltisbergerstr. 18**
**CH-4059 Basel(CH)**
Erfinder: **Schmidt, Dieter, Dr.**
**Redingstr. 20**
**CH-4052 Basel(CH)**

(74) Vertreter: **Lederer, Franz, Dr. et al**
**Lederer, Keller & Riederer Patentanwälte**
**Lucile-Grahn-Strasse 22**
**W-8000 München 80(DE)**

**EP 0 178 450 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 94, Nr. 26, 29 Juni 1981, Columbus, OH (US); M.L.STONE et al., S. 551, Nr. 216784x

AUSTRALIAN JOURNAL OF CHEMISTRY, Band 17, 1964, East Melbourne (AU); D.A.BUCKINGHAM et al., Seiten 325-336

JOURNAL OF INORGANIC AND NUCLEAR CHEMISTRY, Band 41, 1979, Pergamon Press Ltd, Oxford (GB); J.L.KAHL et al., Seiten 495-502

**Beschreibung**

Die vorliegende Erfindung betrifft Rutheniumkomplexe, an die ein immunologisch aktives Material gekoppelt werden kann.

US-A-4293 310 beschreibt ein Fluoreszenzmeßgerat und dafür geeignete o-Phenanthrolin-und 2,2'-Bipyridin-Komplexe des Rutheniums zur Markierung eines Analyten.

Die Rutheniumkomplexe dervorliegenden Erfindung weisen vor der Kopplung die allgemeine Formel

$$Ru^{2+}L_1L_2L_3 \qquad I$$

auf,

worin $L_1$, $L_2$ und $L_3$ gleich oder verschieden sind und je einen bi- oder polycyclischen Liganden mit mindestens zwei stickstoffhaltigen Heterocyclen bedeuten, wobei mindestens einer dieser Liganden mit mindestens einer wasserlöslich machenden Gruppe wie $-SO_3H$ oder-COOH substituiert ist, und wobei mindestens einer dieser Liganden mit mindestens einer reaktiven Gruppe, wie-COOH direkt oder über eine Spacergruppe, substituiert ist, und wobei die Liganden $L_1$, $L_2$ und $L_3$ über Stickstoffatome an das Ruthenium gebunden sind.

Die Liganden $L_1$ und $L_2$ können gleich oder verschieden sein und beispielsweise 2,2'-Bipyridin- , 1,10-Phenanthrolin-, Benzbathophenanthrolin- oder insbesondere Bathophenanthrolingruppen enthalten. Die Liganden $L_1$ und $L_2$ sind vorzugsweise gleich.

Als Gruppen, welche die Liganden wasserlöslich machen, kommen insbesondere Sulfonsäuregruppen in betracht, die vorzugsweise in Form ihrer Salze vorliegen. Besonders bevorzugt sind hierbei die Natriumsalze.

Der Ligand $L_3$ enthält beispielsweise eine 2,2'-Bipyridin-, 1,10-Phenanthrolin- oder insbesondere eine Bathophenanthrolingruppe.

Die eingangs erwähnte Spacergruppe ist vorzugsweise eine Alkylengruppe mit höchstens 8 C-Atomen, die gegebenenfalls $-SO_2-NH-$, oder -COO- aufweisen kann.

Als reaktive Gruppen, an die das immunologisch aktive Material gebunden wird, kommen vorzugsweise -COOH, oder $-SO_2$ Hal Gruppen in Betracht.

Bei Ru-Komplexen mit 3 identischen Liganden $L_1$, $L_2$ und $L_3$ muss der Ligand sowohl eine wasserlöslich machende Gruppe wie auch eine Spacergruppe mit reaktiver Gruppe aufweisen.

Ein hierfür geeigneter Ligand ist die Verbindung der Formel

$[(SO_3Na)(SO_2NH\ CH_2CH_2COOH)batho]$

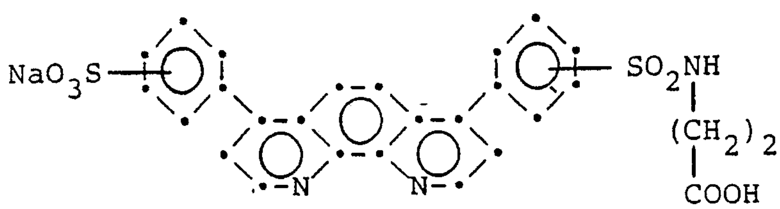

Bei Ru-Komplexen, die zwei verschiedene Ligandtypen aufweisen, kann der eine Ligandtyp die wasserlöslich machende Gruppe oder Gruppen tragen, während der andere Ligandtyp über eine oder mehrere Linkinggruppen (reaktive Gruppe, die über einen Spacer an Heterocyclus gebunden ist) verfügt.

Als Liganden $L_1$ und $L_2$ eignen sich in diesem Falle vorzüglich die Gruppen der Formel

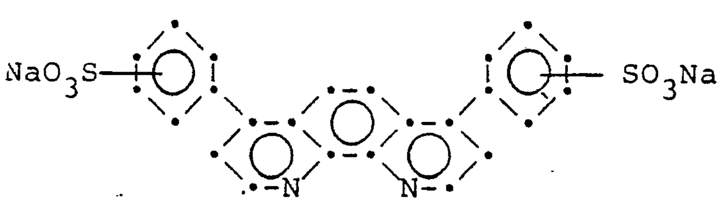

$[(SO_3Na)_2batho]$

und als Ligand $L_3$ vorzugsweise die folgenden Gruppen

$[(SO_3Na)(SO_2NH\ CH_2CH_2COOH)batho]$

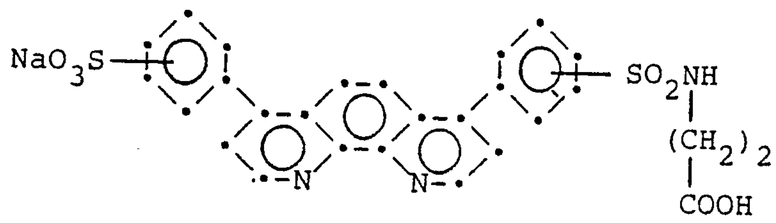

$[(SO_2NH\ CH_2COOH_2)batho]$

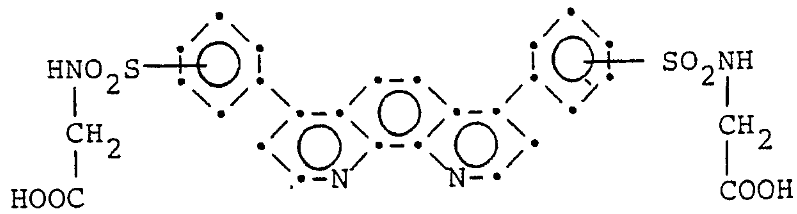

$[(SO_2NH\ CH_2CH_2COOH)_2batho]$

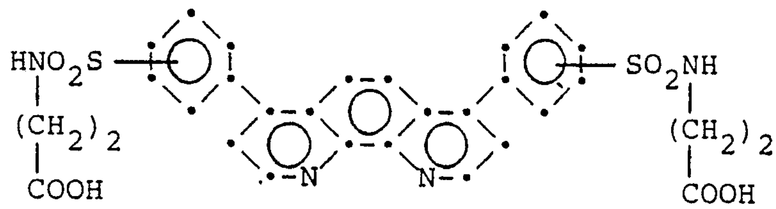

$[(SO_2NH\ CH_2CH_2CH_2COOH)_2batho]$

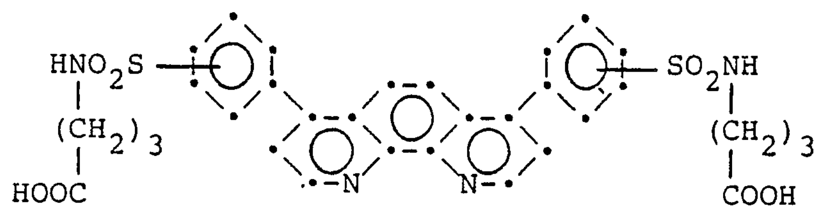

$[(SO_2NH\ CH_2CH_2CH_2CH_2CH_2COOH)_2batho]$

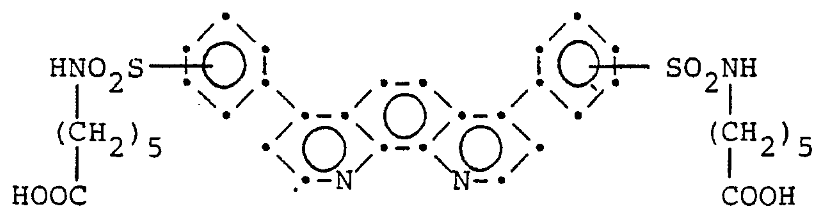

$[(CH_2CH_2CH_2CH_2COOH)batho]$

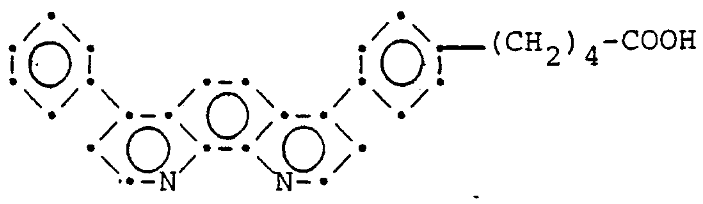

[($CH_2CH_2CH_2CH_2CH_2COOH$)batho]

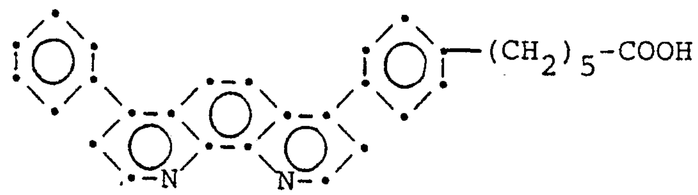

[($CH_2CH_2CH_2CH_2COOH$)benzobatho]

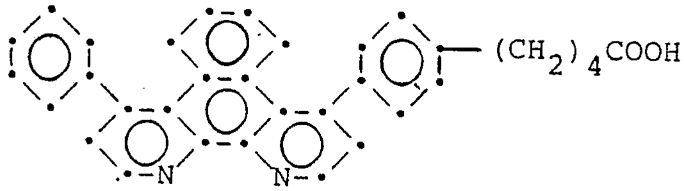

[$(COOH)_2$bpy]

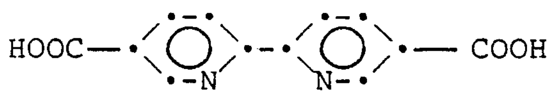

Die Synthese der Liganden $L_3$, welche die Linking Gruppe bzw. Gruppen tragen, erfolgt nach Verfahren, die nachfolgend schematisch beschrieben sind:

a) [($SO_2NH\{CH_2$ $_x$ COO-t-butyl$)_2$batho] und
[($SO_3Na$)($SO_2NH$ $CH_2CH_2COO$-t-butyl)batho

Bei der Synthese dieser Verbindungen geht man vom Dinatrium-Salz der Bathophenanthrolindisulfonsäure aus. Daraus stellt man mit $PCl_5$ zunächst das entsprechende Disulfochlorid her (vgl. F. Muth in "Houben-Weyl, Methoden der Organischen Chemie", Band IX, S. 563, 4. Auflage 1955, G. Thieme Verlag, Stuttgart). Dieses wird anschliessend mit dem t-Butylester einer Aminosäure (wie z.B. $\beta$-Alanin, Glycin, 4-Aminobuttersäure oder 6-Aminocapronsäure) gemäss folgendem Schema zum entsprechenden Sulfonamid umgesetzt (vgl. F. Muth in "Houben-Weyl, Methoden der Organischen Chemie", Band IX, S. 609, 4. Auflage 1955, G. Thieme Verlaug Stuttgart):

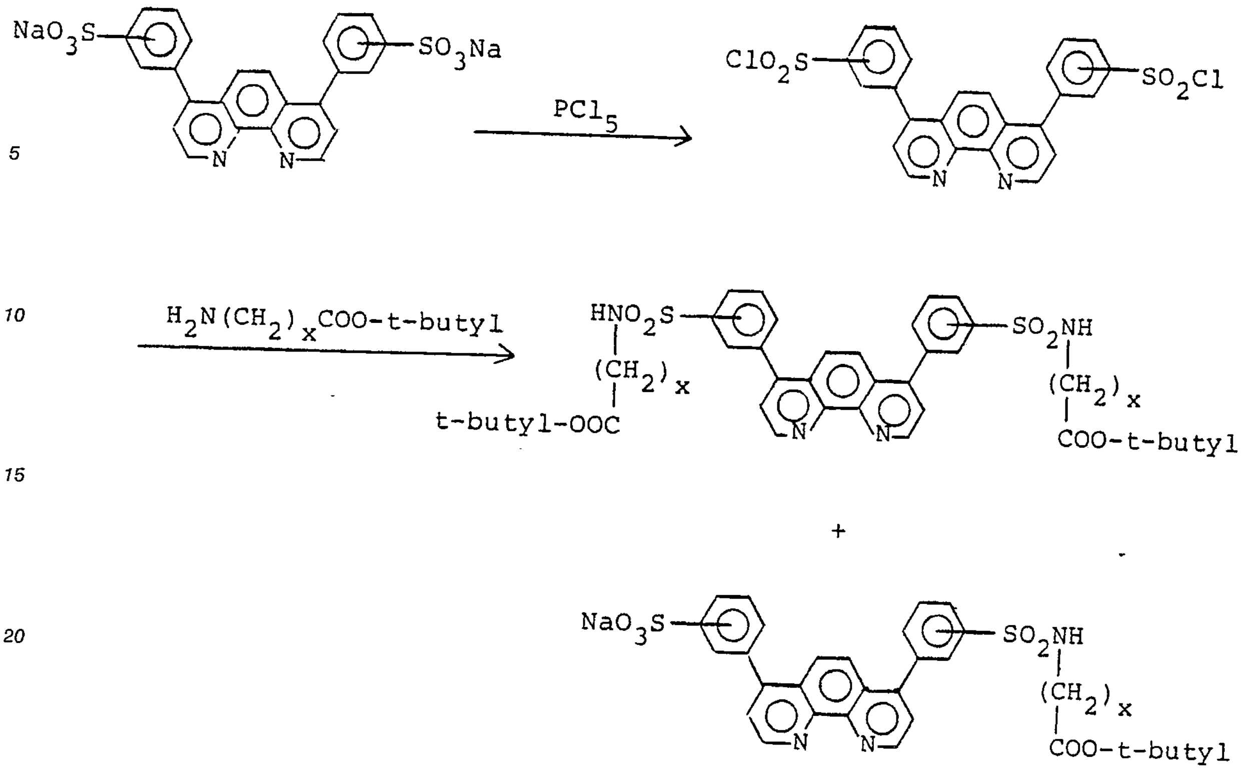


Bei dieser Reaktion entsteht neben dem Disulfonamid als Nebenprodukt immer auch noch das Monosulfonamid.

Die Verseifung der t-Butylester erfolgt erst nach der Synthese der entsprechenden Ru-Komplexe.

b) [($CH_2CH_2CH_2CH_2COOH$)batho] bzw.
[($CH_2CH_2CH_2CH_2CH_2COOH$)batho]

Die Synthese dieser Liganden mit Valeriansäure bzw. Capronsäure als Linking-Gruppe erfolgt mittels Skraup'scher Reaktion (vgl. F.H. Case und P.F. Strohm; J.Org.Chem. 27 , 1641 (1962)) aus 4-Phenyl-8-amino-chinolin (vgl. F.H. Case; J.Org.Chem. 16 , 1541 (1951)) und den p-[β-Chlorpropionyl]-Derivaten der entsprechenden ω-Phenylfettsäuremethylester, wobei die letzteren durch Acylierung des 5-Phenylvaleriansäuremethylester (Fluka) bzw. des 6-Phenylcapronsäuremethylesters (vgl. W.E. Truce und C.E. Olson; J. Amer. Chem.Soc. 75 , 1651 (1953)) mit β-Chlorpropionylchlorid gemäss nachfolgendem Reaktionsschema erhalten werden:

<u>Reaktionsschema</u>

n = 4 and 5

β-Chlor propionyl-chlorid

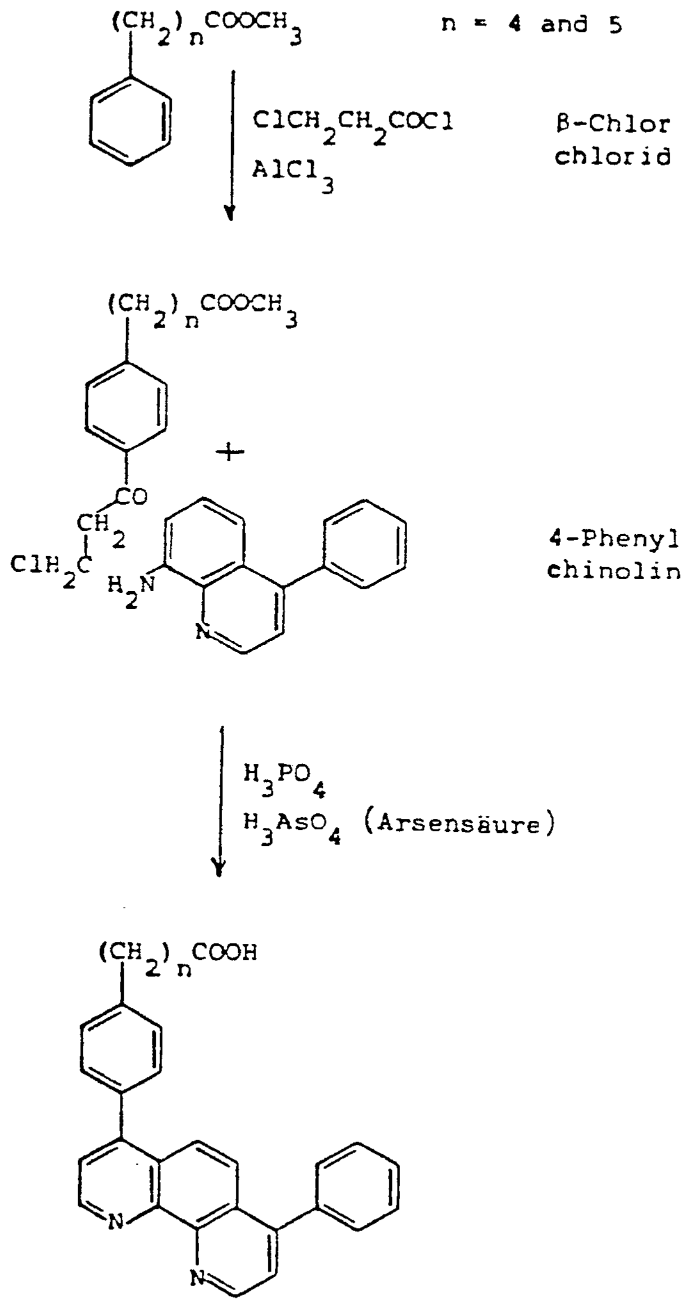


p-(β-Chlorpropionyl)-Derivat der ω-Phenyl-fettsäuremethylester

4-Phenyl-8-aminochinolin

n = 4: 5-[p-(7-Phenyl-1,10-phenanthrolin-4-yl)phenyl]pentansäure ≡[ $(CH_2CH_2CH_2CH_2COOH)$batho]

n = 5: 6-[p-(7-Phenyl-1,10-phenanthrolin-4-yl)phenyl]hexansäure ≡[ $(CH_2CH_2CH_2CH_2CH_2COOH)$batho]

c) [$(CH_2CH_2CH_2CH_2COOH)$benzobatho]

Die Synthese dieser Verbindung (Benzo-bathophenanthrolin-pentansäure) erfolgt über mehrere Stufen aus 2-Amino-3-naphthoesäure gemäss folgendem Schema (vgl. E. Koft und F.H. Case; J.Org.Chem. 27 ,

865 (1962))

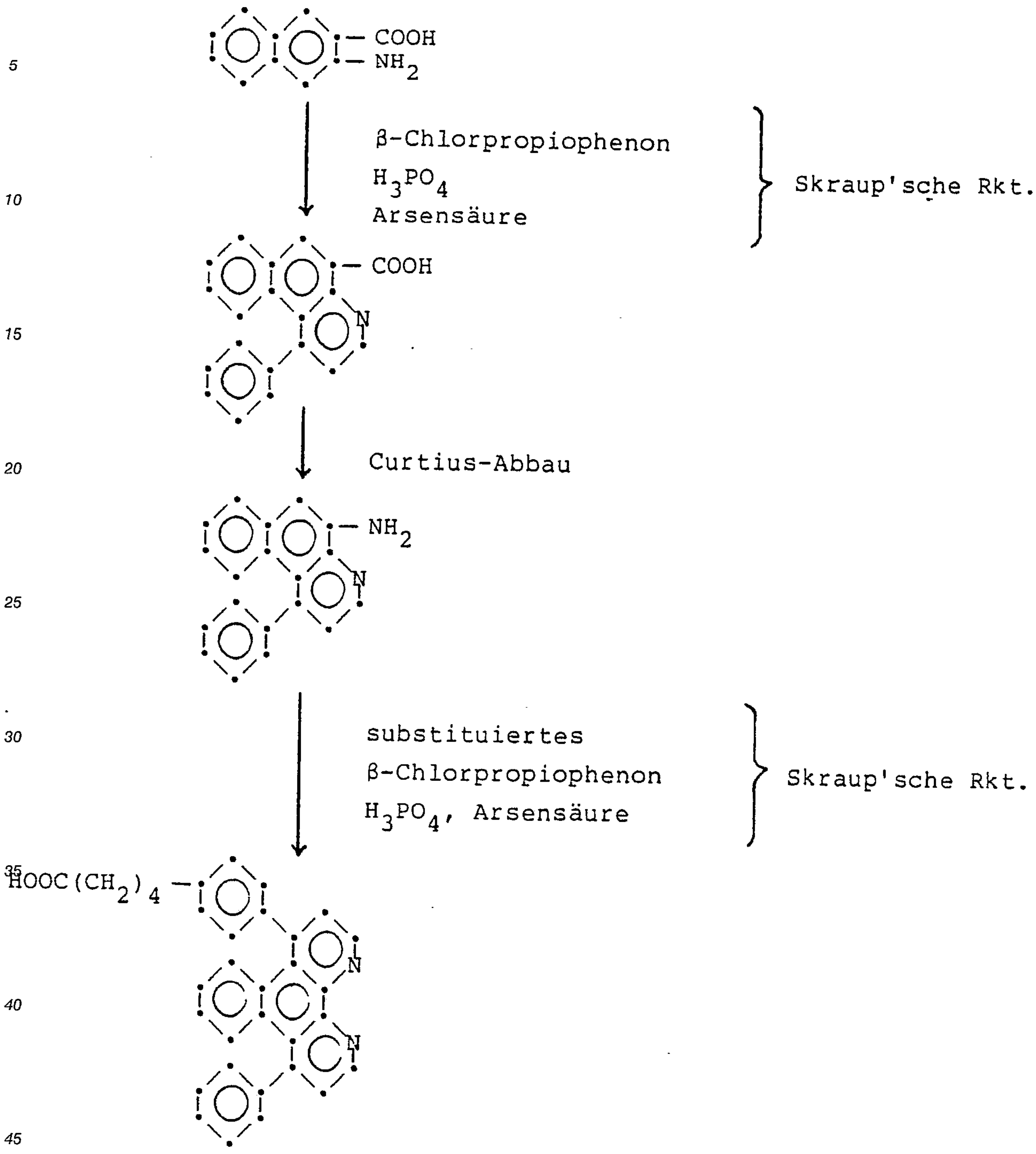


Für die Synthese von Ru-Komplexen mit 3 identischen Liganden werden in der Literatur 2 Verfahren beschrieben:

Nach Braddock und Meyer [J.Am.Chem.Soc. 95 , 3158 (1973)] erhitzt man wasserlösliches Rutheniumtrichlorid zusammen mit dem Liganden für längere Zeit in DMF, wobei sich der Komplex unter teilweiser Zersetzung des Lösemittels bildet.

Bevorzugt verwendet man jedoch das von Lin et al. [J.Am.Chem.Soc. 98 , 6536 (1976)] beschriebene Verfahren. Dabei erhitzt man Kaliumpentachloroaquoruthenat ($K_2Ru\ Cl_5 \cdot H_2O$) in schwach salzsaurer Umgebung mit der 3-fachen stöchiometrischen Menge an Ligand und reduziert anschliessend die Lösung mit Natriumhypophosphit.

Die Herstellung der Rutheniumkomplexe mit 2 verschiedenen Ligandtypen erfolgt nach literaturbekannten Methoden, z.B. gemäss Belser et al. Helv.Chim.Acta 63, 1675 (1980).

Im Hinblick auf die Tatsache, dass bei diesen Komplexen der Ligand $L_3$ von den Liganden $L_1$ und $L_2$

verschieden ist, wird ein stufenweiser Aufbau des Komplexes bevorzugt. (Selbstverständlich könnte der Komplex auch mittels einer statistischen Synthese hergestellt werden, doch ist diese Möglichkeit nicht bevorzugt, da sie schwerer kontrollierbar ist.) Dabei wird in einer ersten Stufe mit wasserlöslichem Rutheniumtrichlorid und der doppelt stöchiometrischen Menge an Ligand $L_1$ bzw. $L_2$ in Dimethylformamid (mit Lithiumchlorid als Katalysator) das Zwischenprodukt $RuL_1L_2Cl_2$ hergestellt. Dieses Zwischenprodukt wird mit dem Liganden $L_3$ anschliessend zum gewünschten Komplex umgesetzt.

Wird als Ligand $L_3$ eine Verbindung verwendet, deren reaktive Gruppe in geschützter Form vorliegt - z.B. als t-Butylester bei den Sulfonamid-Derivaten des Bathophenanthrolins - so erfolgt die Abspaltung dieser Schutzgruppe bevorzugt erst nach der Synthese des entsprechenden Ru-Komplexes.

Die Isolierung und Reinigung der gebildeten Rutheniumkomplexe erfolgt nach herkömmlichen Methoden, durch Umfällen, Säulenchromatographie oder präparative Dickschichtchromatographie.

Als immunologisch aktive Materialien, welche an die vorerwähnten Rutheniumkomplexe gekoppelt sind, kommen insbesondere Antigene, Haptene oder Antikörper (inklusive, z.B. Fab-Fragmente) in Betracht. Als Antikörper können sowohl polyklonale wie monoklonale Antikörper verwendet werden.

Ein besonders bevorzugtes immunologisch aktives Material ist ein Antikörper gegen carcinoembryonales Antigen. Ein weiterhin besonders bevorzugtes immunologisch aktives Material ist ein Antikörper gegen menschliches Choriongonadotropin, sowie ein Antikörper gegen $\alpha$-Interferon.

Die Kopplung des immunologischen Materials an den Rutheniumkomplex erfolgt in an sich bekannter Weise. Eine bevorzugte Kopplungsweise besteht darin, dass man den Rutheniumkomplex und das immunologisch aktive Material mit einem wasserlöslichem Carbodiimidderivat, z.B. mit N-Cyclohexyl-N'-(2-morpholinoäthyl)-carbodiimid -methyl-p-toluolsulfonat behandelt.

Die Rutheniumkomplexe gemäss vorliegender Erfindung lassen sich fluoreszenzspektroskopisch sehr empfindlich nachweisen. Sie sind bestens geeignet als Markermoleküle für hochempfindliche Fluoreszenz-Immuno-Assays. Sie sind insbesondere geeignet für einen zeitaufgelösten Fluoreszenz-Immuno-Assay, wie er beispielsweise in der DT-OS 2628158 beschrieben wird. Durch die Verwendung der Rutheniumkomplexe gemäss vorliegender Erfindung anstelle des häufig verwendeten FITC (Fluoresceinisothiocyanat) kann die Nachweisempfindlichkeit bei Fluoreszenz-Immuno-Assays verbessert werden. Dies ist insbesondere bei der Bestimmung geringer Mengen von Antigenen in Körperflüssigkeiten, wie z.B. Plasma und Serum, von Vorteil. Beispiele für solche Antigene sind z.B. das carcinoembryonale Antigen (CEA), das $\beta$-HCG oder das $\alpha$-Interferon.

Die im Rahmen der vorliegenden Erfindung verwendete Messapparatur für die zeitaufgelöste Fluoreszenzmessung wird im folgenden beschrieben und anhand eines Schemas erläutert.

Eine gepulste Lichtquelle - Farbstofflaser - regt mit Lichtblitzen geeigneter Wellenlänge, $\lambda = 453$ nm, die Messprobe zur Fluoreszenz an, wobei die Blitzdauer $t = 0{,}7$ ns viel kürzer als die Abklingzeit des fluoreszierenden Markers ist. Die Fluoreszenzstrahlung wird dann mit einer Optik durch einen Kantenfilter (Balzers 610), der die Emissionswellenlänge des Markers durchlässt, auf die Photokathode eines Photomultipliers geführt. Die einzelnen detektierten Photonen erzeugen Strompulse, die nach Verstärkung und Normierung digital gezählt werden (Photon-counting-Methode). Ueber eine Photodiode steuert das anregende Blitzlicht gleichzeitig eine Torschaltung, welche nach einer einstellbaren Verzögerungszeit $\delta = 2\ \mu s$ den Zähler startet und nach einer einstellbaren Oeffnungszeit des Messfensters $\Delta t = 3\ \mu s$ den Zählprozess wieder stoppt. Die Verzögerungszeit $\Delta$ wird so gewählt, dass in ihr Streulichteffekte sowie die Backgroundfluoreszenz praktisch vollständig abgeklungen sind. Auf diese Art wird die Anzahl gezählter Pulse proportional zu der Markerfluoreszenzintensität, welche separat vom Background gemessen wird.

<u>Schematische Darstellung der Messapparatur</u>

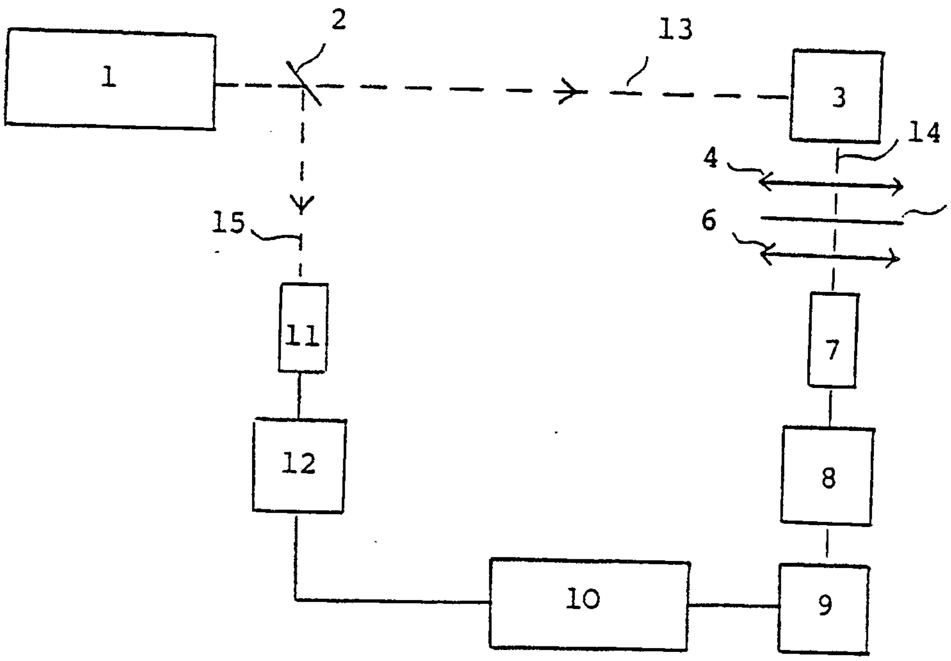


1. Lichtquelle
2. Quarzplatte
3. Probe
4. Sammellinse
5. Kantenfilter
6. Fokussierlinse
7. Photomultiplier
8. Verstärker
9. Diskriminator
10. Zähler
11. Photodiode
12. Torschaltung
13. Anregungs-Lichtblitz
14. Fluoreszenzlicht
15. Lichtblitz zur Steuerung der Torschaltung (Trigger)

Beispiel 1

Herstellung von Bathophenanthrolindisulfochlorid

2,2 g getrocknetes Bathophenanthrolindisulfonsäure-Dinatriumsalz (Fluka) werden mit 3,1 g $PCl_5$ und 750 µl $POCl_3$ in einem 500 ml Rundkolben gut durchmischt. Der Kolben wird, versehen mit einem Rückflusskühler und einem Calciumchloridrohr, 2,5 Stunden lang in einem Oelbad auf 110 °C erhitzt. Absublimiertes $PCl_5$, das sich an den kühleren Stellen des Kolbens niedergeschlagen hat, wird zwischendurch abgekratzt. Anschliessend zieht man am Wasserstrahlvakuum bei 110 °C das nicht umgesetzte $PCl_5$ sowie das $POCl_3$ innerhalb von 5 Stunden vollständig ab.

Nach dem Abkühlen auf Zimmertemperatur behandelt man das rohe Sulfochlorid zunächst kurz mit 150 ml Benzol, die verworfen werden (um Spuren von Verunreinigungen zu entfernen). Anschliessend wird es zweimal mit je 150 ml Chloroform extrahiert, wobei jeweils 1 Stunde bei Zimmertemperatur gerührt wird.

Die vereinigten Chloroformextrakte engt man am Vakuum ein. Das zurückbleibende Sulfochlorid wird dann 4 Stunden lang am Vakuum bei 110 °C getrocknet (Ausbeute: 1,8 g).

Herstellung von Sulfonamidderivaten des Bathophenanthrolins mit dem t-Butylester des β-Alanins

$[(SO_2\text{-}NH\text{-}CH_2CH_2\text{-}COO\text{-}t\text{-}butyl)_2batho]$ und
$[(SO_3Na)(SO_2NH\ CH_2CH_2COO\text{-}t\text{-}butyl)batho]$

1,32 g β-Alanin-t-butylester und 10,5 ml Triäthylamin werden in 50 ml Chloroform aufgelöst. Zu dieser Lösung gibt man unter starkem Rühren innerhalb von 10 Minuten 2,0 g festes Bathophenanthrolindisulfochlorid. Nach 5-stündigem Rühren bei Zimmertemperatur lässt man das Reaktionsgemisch 4 Tage lang im Dunkeln stehen. Anschliessend zieht man bei 40-50° C am Vakuum das Lösemittel sowie das Triäthylamin ab. Zur vollständigen Entfernung des Triäthylamins versetzt man den Rückstand mit 200 ml Chloroform und zieht dieses am Vakuum wieder ab. Dieses Verfahren wird insgesamt fünfmal durchgeführt, bis das Produkt keinen Geruch mehr nach Triäthylamin aufweist.

Die Reinigung des Rückstandes erfolgt durch Chromatographie über $SiO_2$. Sie führt zu 2 Produkten.

a) Isolation des Disulfonamids

$[(SO_2NHCH_2CH_2COO\text{-}t\text{-}butyl)_2batho]$

Mit 8 l Aceton eluiert man dabei zunächst das praktisch reine Disulfonamid. Ausbeute: 900 mg.

b) Isolation des Monosulfonamids

$[(SO_3Na)(SO_2NH\text{-}CH_2CH_2\text{-}COO\text{-}t\text{-}butyl)batho]$

Mit 3 l eines Gemisches aus Chloroform/Methanol/Wasser (7/3/0,5) eluiert man eine weitere Zone, die aufgrund ihrer Fluoreszenzeigenschaften ein Phenanthrolinderivat sein muss. Nach NMR handelt es sich dabei um das Monosulfonamid der Bathophenanthrolindisulfonsäure, das wahrscheinlich durch teilweise Hydrolyse des Bathophenanthrolindisulfochlorids bei der Reaktion hat entstehen können (Ausbeute: 1,2 g Rohprodukt).

Zur weiteren Reinigung löst man das Rohprodukt in 150 ml Chloroform und schüttelt diese Lösung insgesamt dreimal mit je 100 ml Wasser aus. Anschliessend wird das Monosulfonamid noch zweimal umgefällt. Hierzu löst man die Verbindung in 50 ml Methanol/Chloroform (3/1) und fällt sie wieder durch langsames Zutropfen von 200 ml Aether aus. Ausbeute: 360 mg.

Herstellung des Ru-Komplexes [RC-1]

$Ru[(SO_3Na)(SO_2NH\ CH_2CH_2COOH)batho]_3(PF_6)_2$

18,7 mg Kaliumpentachloroaquoruthenat ($K_2RuCl_5{\cdot}H_2O$) werden bei 60° in 2 ml Wasser aufgelöst, dem vorher noch 1 Tropfen 6N HCl zugesetzt worden war. Zu dieser Lösung gab man die 3-fache stöchiometrische Menge an Ligand (95.8 mg tert-Butylester gemäss b) gelöst in 1 ml DMF) und erhitzte das Gemisch 2,5 Stunden lang unter $N_2$ am Rückfluss.

Nach dem Abkühlen der Reaktionslösung wird der entstandene $Ru^{3+}$-Komplex mit 250 µl einer 1 molaren $NaH_2PO_2$-Lösung zum entsprechenden $Ru^{2+}$-Komplex reduziert und dann nochmals für 2 Stunden am Rückfluss gekocht.

Anschliessend filtrierte man die Lösung, versetzte sie mit 900 µl einer 10%igen wässrigen Ammoniumhexafluorophosphatlösung und lässt sie über Nacht bei 4° C stehen. Dabei fällt der Komplex aus.

Nach dem Abnutschen wird er mittels präparativer Dickschichtchromatographie (4 Kieselgel-Platten, Laufmittel $CH_2Cl_2/MeOH/H_2O$ (7:3:0,5) gereinigt.

Eine vordere rote Zone wird isoliert (50 mg reines Produkt) und zur Verseifung mit 5 ml Trifluoressigsäure bei Zimmertemperatur versetzt. Nach 1 Stunde wurde die Trifluoressigsäure am Wasserstrahlvakuum abgezogen.

Beispiel 2

Herstellung von Ru $[(SO_3Na)_2batho]_2Cl_2$

627 mg $RuCl_3{\cdot}3H_2O$, 568 mg LiCl und 3,26 g Bathophenanthrolindisulfonsäure-diNa-Salz (Fluka) werden in 8 ml DMF 6 Stunden lang am Rückfluss gekocht. Nach dem Abkühlen auf Zimmertemperatur versetzt

man die Reaktionslösung langsam mit 60 ml Aceton und lässt sie dann 20 Stunden lang bei 4° C stehen. Dabei fällt das violette Rohprodukt aus. Dieses wird abgenutscht und gut mit Aceton gewaschen. Eine erste Reinigung erfolgt dann durch Umfällen. Hierzu löst man das Rohprodukt in 50 ml Methanol und fällt es anschliessend wieder mit 500 ml Aceton/Aether (1/1) aus. Dieses Verfahren wird wiederholt. Die weitere Reinigung erfolgt durch Chromatographie über $SiO_2$. Mit 5 l Chloroform/Methanol/Aceton (4/3/3) eluiert man 600 mg praktisch reines Produkt (Ausbeute: 600 mg).

Herstellung des gemischten Komplexes. $Ru[SO_3Na)_2batho]_2$ $[(SO_2\text{-}NH\text{-}CH_2\text{-}CH_2\text{-}COO\text{-}t\text{-}butyl)_2batho]Cl_2$

76,2 mg $Ru[(So_3Na)_2batho]_2Cl_2$ werden in einem Gemisch aus 1 ml Wasser und 4 ml Methanol gelöst und mit 41,0 mg $[(SO_2\text{-}NH\text{-}CH_2\text{-}CH_2\text{-}COO\text{-}t\text{-}butyl)_2batho]$ (vgl. Beispiel 1a) (gelöst in 3 ml Chloroform) vermischt. Dieses Reaktionsgemisch wird 3 Stunden lang unter $N_2$ am Rückfluss erhitzt, wobei eine orange-rote Lösung entsteht. Anschliessend wird das Lösemittel durch Einblasen von $N_2$ und leichtem Erwärmen zu etwa 90% abdestilliert. Beim Abkühlen auf Zimmertemperatur fällt ein Teil des Produktes aus. Zur Reinigung löst man das Produkt in 1,5 ml Methanol und 0,5 ml DMF und chromatographiert es über $SiO_2$ mit dem Laufmittel Chloroform/Methanol/Wasser (7/3/0,5) (Ausbeute: 75 mg).

Herstellung des Ru-Komplexes [RC-2] $Ru[SO_3Na)_2batho]_2$ $[(SO_2\text{-}NH\text{-}CH_2\text{-}CH_2\text{-}COOH)_2batho]Cl_2$

Verseifung des t-Butylesters. Hierzu werden 55 mg $Ru[(SO_3Na)_2batho]_2$ $[(SO_2\text{-}NH\text{-}CH_2\text{-}CH_2\text{-}COO\text{-}t\text{-}butyl)_2batho]Cl_2$ in 5 ml Trifluoressigsäure gelöst. Das Reaktionsgemisch lässt man 1 Stunde bei Zimmertemperatur stehen und zieht dann am Wasserstrahlvakuum bei 40° C die Trifluoressigsäure ab.

Das Rohprodukt wurde zunächst durch Säulenchromatographie über $SiO_2$ gereinigt. Laufmittel:

300 ml Chloroform/Aceton/Methanol (4/3/3)
500 ml Chloroform/Methanol/Wasser (50/50/2)
500 ml Chloroform/Methanol/Wasser (50/50/5)
250 ml Chloroform/Methanol/Wasser (50/50/10)
100 ml Methanol/Wasser (10/1)
100 ml Methanol/Wasser (1/1)

Die endgültige Reinigung erfolgt mittels präparativer Dickschichtchromatographie. 40 mg des Ru-Komplexes werden hierzu in 700 µl Wasser gelöst und auf 4 PSC-Platten ($SiO_2$-Platten von Merck) aufgetragen. Ueber Nacht werden diese bei 60° C getrocknet und anschliessend mit dem Laufmittel Chloroform/Methanol/Wasser (50/50/2) chromatographiert. Die Hauptzone wird herausgekratzt und dreimal mit je 40 ml Wasser extrahiert (das Kieselgel wird dabei durch Zentrifugation abgetrennt). Nach dem Einengen werden vom Produkt noch letzte Spuren $SiO_2$ entfernt, indem man es in wenig Methanol löst und das ungelöste Kieselgel abzentrifugiert (Ausbeute: 38 mg).

## Beispiel 3

Herstellung des Bathophenanthrolindisulfonamids mit dem t-Butylester des Glycins. $[(SO_2NH\ CH_2\ COO\text{-}t\text{-}butyl)_2\ batho]$

Diese Sulfonamidsynthese erfolgte analog der in Beispiel 1 beschriebenen.
Ansatz: Glycin-t-butylester Dibenzolsulfonamidsalz (10,3 g), Triäthylamin (28 ml) und Bathophenanthrolindisulfochlorid (4,2 g).
Ausbeute: 4,9 g $[(SO_2NH\ CH_2COO\text{-}t\text{-}butyl)_2batho]$

Herstellung des gemischten Komplexes.

$Ru[(SO_3Na)_2batho]_2[(SO_2NH\ CH_2\ COO\text{-}t\text{-}butyl)_2batho]Cl_2$

3,6 g $Ru[(SO_3Na)_2batho]_2Cl_2$ (siehe Beispiel 2) werden in einem Gemisch aus 160 ml Methanol und

100 ml Wasser gelöst und mit 2,01 g [($SO_2NH$ $CH_2COO$-t-butyl)$_2$batho] (gelöst in 80 ml Methanol) vermischt.

Dieses Reaktionsgemisch wird 5 Stunden lang unter $N_2$ am Rückfluss gekocht, wobei eine tiefrote Lösung entsteht. Nach dem Abkühlen engt man die Reaktionslösung am Vakuum auf etwa 70 ml ein und fällt den gemischten Ru-Komplex dann durch langsames Zutropfen von 1,4 l Aceton aus.

Zur weiteren Reinigung wird das abgenutschte Rohprodukt zweimal umgefällt. Man löst hierzu den Niederschlag in etwa 150 ml $MeOH/H_2O/CH_2Cl_2$ (2:1:0,3) und fällt den Komplex dann wieder aus durch langsames Zutropfen von 1,5 l Aceton.

Zum Schluss wird das Produkt noch zweimal über Kieselgel chromatographiert mit dem Laufmittel $CH_2Cl_2/MeOH/H_2O$ (7:3:0,5). Nach einer weiteren Umfällung analog oben erhält man ein reines Produkt. Ausbeute: 3,0 g rotes Pulver.

Verseifung des t-Butylesters zum label [RC-3]
Ru[($SO_3Na$)$_2$batho]$_2$ [($SO_2NH$ $CH_2COOH$)$_2$batho]$Cl_2$

500 mg Ru[($SO_3Na$)$_2$batho]$_2$[($SO_2NHCH_2COO$-t-butyl)$_2$batho]$Cl_2$ werden in 40 ml Trifluoressigsäure dispergiert. Nach 2,5-stündigem Rühren bei RT wird die Trifluoressigsäure am Vakuum abgezogen. Den Rückstand löst man in einem Gemisch aus 1 ml DMF und 3 ml Wasser. Durch langsames Zutropfen von 500 ml Aceton/MeOH (8:2) fällt man daraus den Komplex wieder aus.

Dieser Umfällprozess wird noch zweimal wiederholt, dann trocknet man das rote Pulver bei 70$^\circ$ C am Vakuum. Ausbeute: 420 mg.

Beispiel 4

Herstellung des Bathophenanthrolindisulfonamids mit dem t-Butylester der 4-Aminobuttersäure

[($SO_2NHCH_2CH_2CH_2COO$-t-butyl)$_2$batho]

Diese Sulfonamidsynthese erfolgte analog der in Beispiel 1 beschriebenen.

Ansatz: 4-Aminobuttersäure-t-butylester (1,57 g) Triäthylamin (17 ml) und Bathophenanthrolindisulfochlorid (2,1 g). Ausbeute: 1,3 g [($SO_2NH$ $CH_2CH_2CH_2COO$-t-butyl)$_2$batho]

Herstellung des gemischten Komplexes

Ru[($SO_3Na$)$_2$batho]$_2$[($SO_2NH$ $CH_2CH_2CH_2COO$-t-butyl)$_2$batho]$Cl_2$

Diese Synthese erfolgte analog der Vorschrift von Beispiel 3.

Ansatz: 1,28 g Ru[($SO_3Na$)$_2$batho]$_2Cl_2$ und 0,775 g [($SO_2NH$ $CH_2CH_2CH_2COO$-t-butyl)$_2$batho]. Ausbeute: 1,65 g rotes Pulver.

Verseifung des t-Butylesters zum label [RC-4]

Ru[($SO_3Na$)$_2$batho]$_2$[($SO_2NH$ $CH_2CH_2CH_2COOH$)$_2$batho]$Cl_2$

Ansatz: Verseifung von 70 mg t-Butylester-Derivat Ru[($SO_3Na$)$_2$batho]$_2$[($SO_2NH$ $CH_2CH_2CH_2$-COO-t-butyl)$_2$ batho]$Cl_2$ analog der Vorschrift von Beispiel 3.

Ausbeute: 50 mg.

Beispiel 5

Herstellung des Bathophenanthrolindisulfonamids mit dem t-Butylester der 6-Aminocapronsäure

[($SO_2NH$ $CH_2CH_2CH_2CH_2CH_2$ COO-t-butyl)$_2$batho]

Diese Sulfonamidsynthese erfolgte analog der in Beispiel 1 beschriebenen.

Ansatz: 6-Aminocapronsäure-t-butylester (2,27 g) Triäthylamin (17 ml) und Bathophenanthrolindisulfochlorid (2,1 g). Ausbeute: 1,8 g [($SO_2$NH $CH_2CH_2CH_2CH_2CH_2$COO-t-butyl)$_2$batho].

Herstellung des gemischten Komplexes

Ru[($SO_3$Na)$_2$batho]$_2$[($SO_2$NH $CH_2CH_2CH_2CH_2CH_2$COO-t-butyl)$_2$batho]$Cl_2$

Diese Synthese erfolgte analog der Vorschrift von Beispiel 3.

Ansatz: 2,3 g Ru[($SO_3$Na)$_2$batho]$_2Cl_2$ und 1,49 g [($SO_2$NH $CH_2CH_2CH_2CH_2CH_2$COO-t-butyl)$_2$batho]. Ausbeute: 2,8 g rotes Pulver.

Verseifung des t-Butylesters zum label [RC-5]

Ru[($SO_3$Na)$_2$batho]$_2$[($SO_2$NH $CH_2CH_2CH_2CH_2CH_2$COOH)$_2$batho]$Cl_2$

Ansatz: Verseifung von 100 mg t-Butylester-Derivat Ru[($SO_3$Na)$_2$batho]$_2$[($SO_2$NH $CH_2CH_2CH_2CH_2CH_2$COO-t-butyl)$_2$batho]$Cl_2$ analog der Vorschrift von Beispiel 3. Ausbeute: 85 mg.

Beispiel 6

Herstellung des gemischten Komplexes

Ru[($SO_3$Na)$_2$batho]$_2$[($SO_3$Na)($SO_2$NH $CH_2CH_2$COO-t-butyl)batho]$Cl_2$

Diese Synthese erfolgte analog der Vorschrift von Beispiel 3.

Ansatz: 102 mg Ru[($SO_3$Na)$_2$batho]$_2Cl_2$ und 51 mg [($SO_3$Na)($SO_2$NH $CH_2CH_2$COO-t-butyl)batho] (siehe Beispiel 1). Ausbeute: 105 mg rotes Pulver.

Verseifung des t-Butylesters zum Label [RC-6]

Ru[$SO_3$Na)$_2$batho]$_2$[($SO_3$Na)($SO_2$NH $CH_2CH_2$COOH)batho]$Cl_2$

Ansatz: Verseifung von 105 mg t-Butylester-Derivat Ru[($SO_3$Na)$_2$batho]$_2$[($SO_3$Na)($SO_2$NH $CH_2CH_2$COO-tbutyl)batho]$Cl_2$ analog der Vorschrift von Beispiel 3. Reinigung erfolgt durch präparative Dickschichtchromatographie (Kieselgel-Platten, Laufmittel $CHCl_3$/MeOH/Aceton (4:3:3)). Ausbeute: 32 mg.

Beispiel 7

Synthese von p-[$\beta$-Chlorpropionyl]-5-phenyl-valeriansäuremethylester

In einem Rührkolben mit Rückflusskühler werden 9,3 g $AlCl_3$, 10 ml $CS_2$ und 1,98 ml $\beta$-Chlorpropionylchlorid vorgelegt. Zu diesem Gemisch lässt man bei Zimmertemperatur innert 5 Minuten 3,61 g 5-Phenylvaleriansäure zutropfen (nachgespült wird mit 2 ml $CS_2$). Das Reaktionsgemisch wird anschliessend noch 15 Minuten weiter gerührt, leicht erwärmt und dann abgekühlt.

Dann pipettiert man das Reaktionsgemisch in eine gerührte Mischung von Eis, Wasser und Aether. Die organische Phase wäscht man mit Bicarbonatlösung und Wasser neutral. Nach dem Trocknen über $MgSO_4$ zieht man das Lösemittel ab. Dabei erhält man 5,03 g kristallines Produkt.

Synthese von 5-[p-(7-Phenyl-1,10-phenanthrolin-4-yl)phenyl]-pentansäure

[($CH_2CH_2CH_2CH_2$COOH)batho]

In einem Rührkolben werden unter Argon 2,54 g 4-Phenyl-8-aminochinolin, 11,5 ml $H_3PO_4$ (85%ig) und 2,3 ml Arsensäurelösung (80%ige $H_3AsO_4$) vorgelegt. Zum Lösen des Chinolins erhitzt man das Gemisch auf 120° C. Dann fügt man innerhalb von 5 Minuten noch 4,56 g p-[$\beta$-Chlorpropionyl]-5-phenylvaleriansäu-

remethylester hinzu. Anschliessend heizt man das Reaktionsgemisch unter Rühren innerhalb von 10 Minuten auf 140 °C auf und lässt es auf dieser Temperatur noch 1 Stunde.

Zur Aufarbeitung kühlt man das Reaktionsgemisch ab und pipettiert es in ein gerührtes Gemisch von 50 ml Wasser und 125 ml $CH_2Cl_2$, das gut mit Eis gekühlt wird. Durch Zufügen von Natronlauge bringt man den pH-Wert auf 5. Dabei geht die Substanz in die organische Phase über.

Nach dem Abdampfen des Lösemittels erhält man ein Rohprodukt, das neben dem Methylester auch noch freie Säure enthält (die Reaktionsbedingungen wirken hydrolysierend). Durch Behandlung mit Diazomethan (in Aether/Methanol) wird das Gemisch wieder vollständig in den Methylesterz überführt.

Nach zweimaliger Chromatographie mit Essigester an Alox III (mit 1,5% $H_2O$ noch zusätzlich desaktiviert) erhält man 3,022 g Methylester.

Zur Verseifung löst man diesen in 20 ml Aethanol auf, versetzt die Lösung mit Natronlauge (0,950 g NaOH gelöst in 5 ml $H_2O$) und erhitzt sie unter Argon für 2 Stunden auf 80 °C. Die abgekühlte Lösung wird in ein $CH_2Cl_2/H_2O$ Gemisch gegeben, dann der pH-Wert der Lösung mit 85%iger $H_3PO_4$ auf 4-5 eingestellt und anschliessend das Produkt extrahiert.

Das eingeengte $CH_2Cl_2$-Extrakt wird aus Benzol umkristallisiert, dabei erhält man 1,35 g Produkt. Aus der eingeengten Mutterlauge erhält man durch Umkristallisieren aus Aethanol noch weitere 0,338 g Produkt.

Gesamtausbeute 1,688 g Säure (kristallisiert ohne Lösemittel), Fpkt. 234-235 °C.

Herstellung des Ru-Komplexes [RC-7]

$Ru[(SO_3Na)_2batho]_2\ [(CH_2CH_2CH_2CH_2\text{-}COOH)batho]Cl_2$

Zu einer Lösung von 102,4 mg $Ru[(SO_3Na)_2batho]_2\text{-}Cl_2 \cdot 2H_2O$ in 4 ml Methanol und 8 ml Wasser gibt man 34,6 mg 5-[p-(7-Phenyl-1,10-phenanthrolin-4-yl)phenyl]-pentansäure aufgelöst in 2 ml Methanol. Dieses Gemisch wird 3 Stunden lang unter $N_2$ am Rückfluss erhitzt, wobei eine orange-rote Lösung entsteht.

Anschliessend entfernt man den grössten Teil des Lösungsmittels durch Einblasen von $N_2$. Der Rest wird dann vollends mit dem Rotationsverdampfer abgezogen.

Die Reinigung des Rohproduktes erfolgt mittels präparativer Dickschichtchromatographie. Der Komplex wird hierzu in wenig Wasser aufgelöst und auf 6 PSC-Platten ($SiO_2$-Platten von Merck) aufgetragen. Nach dem Trocknen der Platten bei 70° chromatographiert man mit dem Laufmittel Chloroform/Aceton/Methanol (4/3/3).

Das mit Wasser extrahierte Hauptzonenprodukt wurde noch dreimal einer präparativen Dickschichtchromatographie unterworfen, wobei folgende Laufmittel verwendet wurden: Aceton/Wasser (9/1), dann Aceton/Wasser (8,5/1,5) und schliesslich Chloroform/Methanol/Wasser (50/50/2).

Zur Entfernung von letzten Spuren $SiO_2$ löst man das extrahierte Produkt in wenig Methanol und zentrifugiert das ungelöste Kieselgel ab. Aus 1 ml methanolischer Lösung wurde dann das Produkt mit 50 ml Aceton ausgefällt. Ausbeute: 42,6 mg (rotes Pulver).

## Beispiel 8

Synthese von p-[β-Chlorpropionyl]-6-phenyl-capronsäuremethylester

In einem Rührkolben mit Rückflusskühler werden 17,2 g $AlCl_3$, 18,5 ml $CS_2$ und 3,6 ml β-Chlorpropionylchlorid vorgelegt. Zu diesem Gemisch lässt man bei Zimmertemperatur innert 10 Minuten 7,06 g 6-Phenylcapronsäuremethylester zutropfen (nachgespült wird mit 4 ml $CS_2$). Das Reaktionsgemisch wird anschliessend noch 15 Minuten weiter gerührt, leicht erwärmt und dann abgekühlt.

Dann pipettiert man das Reaktionsgemisch in eine gerührte Mischung von Eis, Wasser und Aether. Die organische Phase wäscht man mit Bicarbonatlösung und Wasser neutral. Nach dem Trocknen über $MgSO_4$ zieht man das Lösungsmittel ab. Dabei erhält man 10,2 g kristallines Produkt.

Synthese von 6 -[p-(7-Phenyl-1,10-phenanthrolin-4-yl)-phenyl]-hexansäure

$[(CH_2CH_2CH_2CH_2CH_2COOH)batho]$

In einem Rührkolben werden unter Argon 5,90 g 4-Phenyl-8-aminochinolin, 27 ml $H_3PO_4$ (85%ig) und 5,35 ml Arsensäurelösung (80%ige $H_3AsO_4$) vorgelegt. Zum Lösen des Chinolins erhitzt man das Gemisch

auf 120° C. Dann fügt man innerhalb von 5 Minuten noch 11,15 g p-[β-Chlorpropionyl]-6-phenylcapronsäuremethylester hinzu. Anschliessend erhitzt man das Reaktionsgemisch unter Rühren innerhalb von 10 Minuten auf 140° C und lässt es auf dieser Temperatur noch 1 Stunde.

Zur Aufarbeitung kühlt man das Reaktionsgemisch ab und pipettiert es in ein gerührtes Gemisch von 100 ml Wasser und 250 ml $CH_2Cl_2$, das gut mit Eis gekühlt wird. Durch Zufügen von Natronlauge (etwa 2/3 einer Lösung aus 45 g NaOH in 200 ml Wasser) bringt man dann den pH-Wert auf 5. Dabei geht die Substanz in die organische Phase über.

Nach dem Abdampfen des Lösemittels erhält man 15,76 g dickflüssiges Oel, das neben dem Methylester auch noch freie Säure enthält (die Reaktionsbedingungen wirken hydrolysierend). Durch Behandlung mit Diazomethan (in Aether/Methanol) wird das Gemisch wieder vollständig in den Methylester überführt. Dieser wird mit Essigester an 350 g Alox III (mit 1% $H_2O$ noch zusätzlich desaktiviert) chromatographiert (Fraktionen à 120 ml). Reines Produkt enthalten die Fraktionen 5 bis 9.

Die Fraktionen 3, 4 und 10 sind dagegen noch verunreinigt. Sie werden nochmals mit Essigester über Aluminiumoxid chromatographiert (wie vorher beschrieben). Aus den reinen Fraktionen der beiden Chromatographien erhält man nach dem Abziehen des Lösemittels 6,979 g Methylester.

Zur Verseifung löst man den Ester in 45 ml Aethanol auf, versetzt diese Lösung mit Natronlauge (2,24 g NaOH gelöst in 11 ml $H_2O$) und erhitzt sie unter Argon für 2 Stunden auf 80° C. Dann zieht man am Rotationsverdampfer das Aethanol ab und nimmt den Rückstand in einem Gemisch von $CH_2Cl_2$/Wasser auf. Mit 3,75 ml $H_3PO_4$ 85%ig wird der pH-Wert auf ca. 3 eingestellt und dann das Produkt extrahiert.

Das Extrakt wird neutral gewaschen, getrocknet und bis auf 300 ml eingeengt. Nach dem Zusatz von insgesamt 3,5 g Norit SX-3 rührt man die Lösung 40 Minuten lang, filtriert sie und engt sie bis auf wenig $CH_2Cl_2$ ein. Nun gibt man 20 ml Benzol hinzu und lässt die Substanz während 48 Stunden bei Zimmertemperatur auskristallisieren.

Durch Abnutschen, Waschen mit Benzol und Trocknen im Exsikkator erhält man 5,393 g Produkt, das im Kristall noch Benzol enthält.

Das Benzol-freie Produkt hat einen Fpkt. von 195°.

Herstellung des Ru-Komplexes [RC-8]

$Ru[(SO_3Na)_2batho]_2[(CH_2CH_2CH_2CH_2CH_2\text{-}COOH)batho]Cl_2$

Zu einer Lösung von 384 mg $Ru[(SO_3Na)_2batho]_2\text{-}Cl_2\cdot 2H_2O$ in 20 ml Methanol und 5 ml Wasser gibt man 134,2 mg 6-[p-(7-Phenyl-1,10-phenanthrolin-4-yl)phenyl]-hexansäure aufgelöst in 5,7 ml Methanol. Dieses Gemisch wird 3 Stunden lang unter $N_2$ am Rückfluss erhitzt, wobei eine orange-rote Lösung entsteht.

Anschliessend entfernt man den grösstem Teil des Lösemittels durch Einblasen von $N_2$. Der Rest wird dann vollends mit dem Rotationsverdampfer abgezogen.

Die Reinigung des Produktes erfolgt zunächst durch zweimalige Säulenchromatographie über $SiO_2$ mit dem Laufmittel Chloroform/Methanol/Wasser (7/3/0,5). Anschliessend wird der Komplex noch mittels präparativer Dickschichtchromatographie gereinigt ($SiO_2$-Platten von Merck). Laufmittel: Chloroform/Methanol/Wasser (7/3/0,5).

Die Hauptzone wird nach dem Herauskratzen mit Methanol extrahiert. Ausbeute: 162 mg (rotes Pulver).

Beispiel 9

Herstellung der Benzobathophenanthrolinyl-pentansäure

$[(CH_2CH_2CH_2CH_2COOH)benzobatho]$

Die Synthese dieser Verbindung erfolgte aus 2-Amino-3-naphthoesäure mittels Skraup'scher Reaktion analog dem in Beispiel 7 angegebenen Verfahren für $[(CH_2CH_2CH_2CH_2COOH)batho]$. Gesamtausbeute: 1,07 g.

Herstellung des Ru-Komplexes [RC-9]

$Ru[(SO_3Na)_2batho]_2\ [(CH_2CH_2CH_2CH_2COOH)benzobatho]Cl_2$

Diese Synthese erfolgte analog der Vorschrift von Beispiel 3.
Ansatz:1,28 g $Ru[(SO_3Na)_2batho]_2Cl_2$ und 0,561 g $[(CH_2CH_2CH_2CH_2COOH)benzobatho]$. Ausbeute: 1,1 g rotes Pulver.

Beispiel 10

Herstellung des Ru-Komplexes [RC-10]

$Ru[(SO_3Na)_2batho]_2[(COOH)_2bpy]Cl_2$
Diese Synthese erfolgte analog der Vorschrift von Beispiel 3.

| | |
|---|---|
| Ansatz: | 256 mg $Ru[(SO_3Na)_2batho]_2Cl_2$,<br>49 mg 4,4'-Dicarboxy-2,2'-bipyridin und<br>60 mg $NaHCO_3$ (zum Solubilisieren des Bipyridins)<br>in 25 ml $MeOH/H_2O$ (1:2). |
| Ausbeute: | 290 mg rotes Pulver. |

Beispiel 11

Markierung von anti-CEA mit dem Ru-Komplex [RC-2]

Die Kopplung des gemischten Ru-Komplexes [RC-2] an anti-CEA erfolgte mit Hilfe des wasserlöslichen Carbodiimid-Derivates N-Cyclohexyl-N'-(2-morpholinoäthyl)-carbodiimid -methyl-p-toluolsulfonat. Da der Komplex über zwei reaktive Gruppen pro Molekül verfügt, wird molar die doppelte Menge an Carbodiimid verwendet.

Für die Kopplungsreaktion stellt man die folgenden Stammlösungen her:

1) 4,00 mg/ml Ru-Komplex [RC-2] in Wasser bei pH 4,5
2) 2,17 mg/ml anti-CEA vom Kaninchen (DAKO Code Nr. A 115, Lot 112 B) in 200 mM $NaHCO_3$; pH 8,5.

136 µl Stammlösung 1 werden mit 264 µl Wasser pH 4,5 (eingestellt mit HCl) verdünnt. Dazu gibt man 0,27 mg N-Cyclohexyl-N'-(2-morpholinoäthyl)-carbodiimid -methyl-p-toluolsulfonat und durchmischt kurz am Vortex. Nach 2 Minuten fügt man 400 µl von der anti-CEA Stammlösung 2 hinzu und durchmischt wieder gut am Vortex. Durch die Zugabe von wenig 1N HCl wird der pH-Wert auf 8,5 eingestellt. Dann lässt man das Reaktionsgemisch 17 Stunden lang bei Zimmertemperatur im Dunkeln stehen.

Zur Abtrennung des markierten anti-CEAs wurden 500 µl des Reaktionsgemisches über eine Säule (Länge 30 cm, Durchmesser 9 mm) mit Acrylamid Gel AcA-54 von LKB chromatographiert (Laufmittel; 150 mM NaCl, 10 mM Na-phosphat, 0,02% $NaN_3$, pH 7,0). Die Fraktionen mit dem höchsten Gehalt an markiertem anti-CEA wurden vereinigt - insgesamt 5,2 ml. In dieser Lösung wurde der Gehalt an anti-CEA und an Ru-Komplex UV-spektroskopisch bestimmt (aus der optischen Dichte bei 278 nm - Absorption von anti-CEA und Ru-Komplex, sowie aus der optischen Dichte bei 445 nm - Absorption des Ru-Komplexes allein). Es wurden dabei folgende Konzentrationen erhalten:

$0,66 \times 10^{-6}$M/l Ru-Komplex
$0,58 \times 10^{-6}$M/l anti-CEA.
Dies entspricht einem Markierungsgrad von 1,1.

Beispiel 12

Durchführung eines Fluoreszenzimmunoassays mit CEA Standards

Zur quantitativen Bestimmung von CEA-Standards wird ein Sandwichtest mit einem monoklonalen CEA-Antikörper und einem polyklonalen CEA-Antikörper (markierte Antikörper von DAKO) wie folgt durchgeführt:
a) In die erforderliche Anzahl Teströhrchen (10 x 75 mm) werden je 0,250 ml CEA-Standardlösung (0 ng/ml CEA; 2,5 ng/ml CEA; 5 ng/ml CEA; 10 ng/ml CEA und 20 ng/ml CEA in 150 mM NaCl, 10 mM Na-Phosphat

mit 20 g/l Rinderserumalbumin) pipettiert, je eine mit monoklonalem anti-CEA sensibilisierte Polystyrolkugel (Durchmesser 6,5 mm) zugefügt und bei 37° C während 24 Stunden inkubiert. Anschliessend werden die Polystyrolkugeln dreimal mit je 2-5 ml destilliertem Wasser gewaschen und dann in Teströhrchen transferiert, die je 0,250 ml Pufferlösung mit 1 x $10^{-8}$ M/l markiertem Kaninchen anti-CEA enthalten (Markierungsgrad 1,1).

Nach einer 24-stündigen Inkubation bei 37° C werden die Kugeln wieder dreimal mit je 2-5 ml destilliertem Wasser gewaschen und anschliessend in Teströhrchen mit je 2 ml Schwefelsäure (0,09N) transferiert. Nach 30 Minuten pipettiert man die Schwefelsäure-Lösung in Messküvetten und misst den Gehalt an Ru-Komplex fluoreszenzspektroskopisch (Anregungswellenlänge 453 nm, Emissionswellenlänge 612 nm).

Die Messung erfolgte mit der früher beschriebenen Apparatur unter Verwendung eines Kantenfilters B 610 von Balzers, einer zeitlichen Verzögerung der Fluoreszenzmessung von 2 µsec (bezogen auf den Anregungspuls) und einer Oeffnung des Messfensters von 3 µsec.

In der Tabelle I sind die Werte einer CEA-Bestimmung aufgeführt, die mit einer Reihe von CEA-Standards von Roche erhalten wurden.

Die Empfindlichkeit bei diesem zweistufigen Test beträgt 60 pg/ml CEA.

Der CEA Test konnte auch in einem Eintopfverfahren gemäss folgendem Verfahren durchgeführt werden.

b) In die erforderliche Anzahl Teströhrchen (10 x 75 mm) gibt man je 0,125 ml CEA-Standardlösung (0 ng/ml CEA; 2,5 ng/ml CEA; 5 ng/ml CEA; 10 ng/ml CEA; 20 ng/ml CEA in fötalem Kälberserum) sowie je 0,125 ml Lösung mit 2 x $10^{-8}$ M/l Kaninchen anti-CEA, das mit dem Ru-Komplex markiert ist (Markierungsgrad 1,25; Verdünnungspuffer pH 7,1 enthält 0,1 M/l Tris, 20% fötales Kälberserum 0,05% Timerosal und 0,02% Tween 20). Dann fügt man noch je eine Polystyrolkugel (Durchmesser 6,5 mm sensibilisiert mit monoklonalem anti-CEA) hinzu und inkubiert bei 37° C während 24 Stunden.

Anschliessend werden die Kugeln dreimal mit je 2-5 ml destilliertem Wasser gewaschen und dann in Teströhrchen mit je 2 ml Schwefelsäure (0,09N) transferiert. Nach 30 Minuten pipettiert man die Schwefelsäure-Lösung in Messküvetten und misst den Gehalt an Ru-Komplex fluoreszenzspektroskopisch (die Messbedingungen sind identisch mit jenen von Teil a)).

Die Resultate dieser CEA-Bestimmung sind in Tabelle II zusammengestellt. Bei den Eintopfverfahren findet man eine Empfindlichkeit von 430 pg/ml CEA.

Tabelle I

Fluoreszenzspektroskopische Bestimmung von CEA-Standards (zweistufiges Verfahren)

| Konzentration an CEA (Roche Standard Lösungen) | Rel. Fluoreszenzintensität |
|---|---|
| 0 ng/ml CEA | 0,071 |
| 2,5 ng/ml CEA | 0,532 |
| 5 ng/ml CEA | 1,041 |
| 10 ng/ml CEA | 1,846 |
| 20 ng/ml CEA | 4,121 |

Tabelle II

Fluoreszenzspektroskopische Bestimmung von CEA-Standards (Eintopf-Verfahren)

| Konzentration an CEA (Roche Standard Lösungen) | Rel. Fluoreszenzintensität |
|---|---|
| 0 ng/ml CEA | 0,27 |
| 2,5 ng/ml CEA | 1,21 |
| 5 ng/ml CEA | 2,66 |
| 10 ng/ml CEA | 5,13 |
| 20 ng/ml CEA | 9,74 |

Beispiel 13

Markierung von anti-HCG

Die Markierung von anti-HCG mit dem Ru-Komplex [RC-2] erfolgte auf gleiche Art und Weise wie jene von anti-CEA gemäss Beispiel 11.

Für die Kopplungsreaktion stellt man die folgenden Stammlösungen her:

1) 4,00 mg/ml Ru-Komplex [RC-2] in Wasser bei pH 4,5

2) 10,83 mg/ml anti-HCG vom Kaninchen (DAKO Code Nr. A 231 Lot 032 A) in 200 mM $NaHCO_3$; pH 8.5.

150 µl Stammlösung 1 werden mit 250 µl Wasser pH 4,5 (eingestellt mit HCl) verdünnt. Zu dieser Lösung gibt man 0,30 mg N-Cyclohexyl-N'-(2-morpholinoäthyl)-carbodiimid -methyl -p-toluolsulfonat und durchmischt kurz am Vortex. Nach 2 Minuten fügt man die anti-HCG-Lösung (222 µl Stammlösung 2 verdünnt mit 178 µl einer 200 mM $NaHCO_3$-Lösung pH 8,5) hinzu und durchmischt gut am Vortex. Mit wenig HCl wird der pH-Wert auf 8,5 eingestellt. Dann lässt man das Reaktionsgemisch 17 Stunden lang bei Zimmertemperatur im Dunkeln stehen.

Zur Abtrennung des markierten anti-HCGs werden 500 µl des Reaktionsgemisches über eine Säule (Länge 30 cm, Durchmesser 9 mm) mit Acrylamid Gel AcA-54 (von LKB) chromatographiert (Laufmittel: 150 mM NaCl, 10 mM Na-Phosphat, 0,02% $NaN_3$, pH 7,0). Die Fraktionen mit dem höchsten Gehalt an markiertem anti-HCG werden vereinigt - insgesamt 5,2 ml. In dieser Lösung wird der Gehalt an anti-HCG und an Ru-Komplex UV-spektroskopisch bestimmt. Es werden dabei folgende Konzentrationen erhalten:

$2{,}01 \times 10^{-6}$ M/l Ru-Komplex

$2{,}04 \times 10^{-6}$ M/l anti-HCG

Dies entspricht einem Markierungsgrad von 1,0.

Beispiel 14

Durchführung eines Fluoreszenzimmunoassays mit β-HGC Standards

Zur quantitativen Bestimmung von β-HCG-Standards wird ein Sandwichtest mit einem momoklonalen β-HCG-Antikörper und einem polyklonalen HCG-Antikörper (markierte Antikörper von DAKO) wie folgt durchgeführt:

In die erforderliche Anzahl Teströhrchen (10 x 75 mm) gibt man je 0,050 ml β-HCG-Standardlösung (0 mIU/ml; 10 mIU/ml; 25 mIU/ml; 50 mIU/ml; 100 mIU/ml; 200 mIU/ml), 0,050 ml Lösung mit $5{,}12 \times 10^{-8}$ M/1 anti-HCG, das mit dem Ru-Komplex markiert ist (Markierungsgrad 1,0; Verdünnungspuffer pH 7,1 enthält 0,1 M/l Tris, 20% fötales Kälberserum, 0,05% Thimerosal und 0,02% Tween 20) sowie je 0,150 ml Pufferlösung (150 mM NaCl, 10 mM Na-Phosphat mit 20 g/l Rinderserumalbumin). Dann fügt man noch je eine Polystyrolkugel (Durchmesser 6,5 mm sensibilisiert mit monoklonalem anti-β-HCG) hinzu und inkubiert bei 37° C während 16 Stunden.

Anschliessend werden die Kugeln dreimal mit je 2-5 ml destilliertem Wasser gewaschen und dann in

Teströhrchen mit je 2 ml Schwefelsäure (0,09N) transferiert. Nach 30 Minuten pipettiert man die Schwefelsäure-Lösung in Messküvetten und misst den Gehalt an Ru-Komplex fluoreszenzspektroskopisch (die Messbedingungen sind identisch mit jenen von Beispiel 12).

Die Resultate dieser β-HCG-Bestimmung sind in der Tabelle III zusammengestellt. Bei diesem Eintopfverfahren findet man eine Empfindlichkeit von 2,2 mIU/ml β-HCG.

Tabelle III

Fluoreszenzspektroskopische Bestimmung von β-HCG-Standards (Eintopf-Verfahren)

| Konzentration an ß-HCG (Roche Standard Lösungen) | Rel. Fluoreszenzintensität |
|---|---|
| 0 mIU/ml | 0,44 |
| 10 mIU/ml | 0,34 |
| 25 mIU/ml | 0,80 |
| 50 mIU/ml | 1,56 |
| 100 mIU/ml | 2,78 |
| 200 mIU/ml | 5,81 |

Beispiel 15

Markierung von anti-α-Interferon

Die Markierung von monoklonalem anti-α-Interferon (von Roche Diagnostica) mit dem Ru-Komplex [RC-2] erfolgte auf gleiche Art und Weise wie jene von anti-CEA gemäss Beispiel 11.

Für die Kopplungsreaktion stellt man die folgenden Stammlösungen her:

1) 3,75 mg Ru-Komplex [RC-2] in Wasser bei pH 4,5

2) 6,0 mg/ml monoklonales anti-α-Interferon in 200 mM $NaHCO_3$; pH 8.5.

Zu 400 µl Stammlösung 1 gibt man 0,81 mg vom wasserlöslichen Carbodiimid-Derivat und durchmischt kurz am Vortex.

Nach 2 Minuten fügt man 400 µl von der anti-α-Interferon-Stammlösung 2 hinzu und durchmischt wiederum kurz am Vortex. Dann lässt man das Reaktionsgemisch 17 Stunden lang bei Zimmertemperatur im Dunkeln stehen.

Zur Abtrennung des markierten anti-α-Interferons werden 500 µl des Reaktionsgemisches über eine Säule (Länge 30 cm, Durchmesser 9 mm) mit Acrylamid-Gel AcA-54 von LKB chromatographiert (Laufmittel: 150 mM NaCl, 10 mM Na-Phosphat, 0,02% $NaN_3$, pH 7,0).

Die Fraktion mit dem höchsten Gehalt an markiertem anti-α-Interferon werden vereinigt - insgesamt 5,2 ml. In dieser Lösung wird der Gehalt an anti-α-Interferon und an Ru-Komplex UV-spektroskopisch bestimmt.

Man erhält dabei folgende Konzentrationen:

$11{,}5 \times 10^{-6}$ M/l Ru-Komplex

$1{,}8 \times 10^{-6}$ M/l anti-α-Interferon (monoklonal).

Dies entspricht einem Markierungsgrad MG von 6,4.

Beispiel 16

Durchführung eines Fluoreszenzimmunoassays mit α-Interferon Standards

Zur quantitativen Bestimmung von Interferon-rαA Standards (recombinant leucocyte interferon) wird ein von Roche Diagnostica als EIA entwickelter Sandwichtest verwendet. Anstelle des Enzym-markierten zweiten Antikörpers benutzt man jedoch monoklonales anti-Interferon, das mit dem Ru-Komplex [RC-2] markiert ist (Markierungsgrad 6,4).

Die quantitative Bestimmung der verschiedenen Interferon-rαA Standards erfolgte nach folgendem Verfahren (einstufiger Test):

In die erforderliche Anzahl Teströhrchen (10 x 75 mm) gibt man je 0,100 ml Interferon-rαA Standard-Lösung (0 U/ml r IFNαA; 25 U/ml r IFNαA; 50 U/ml rIFNαA; 100 U/ml r IFNαA; 150 U/ml r IFNαA; 200 U/ml r IFNαA in Normal-Human-Serum mit Thimerosal) sowie je 0,500 ml Lösung mit 5,26 x $10^{-10}$ M/l monoklonalem anti-α-Interferon, das mit dem Ru-Komplex [RC-2] markiert ist (Markierungsgrad 6,4; Puffersystem: 150 mM NaCl, 10 mM Na-Phosphat pH 7,5). Dann fügt man noch je eine Polystyrolkugel (Durchmesser 6,5 mm; sensibilisiert mit monoklonalem anti-Interferon) hinzu und inkubiert bei Zimmertemperatur (26° C) während 24 Stunden.

Anschliessend werden die Kugeln dreimal mit je 2-5 ml destilliertem Wasser gewaschen und dann in Teströhrchen mit je 2 ml Schwefelsäure (0,09N) transferiert. Nach 30 Minuten pipettiert man die Schwefelsäure-Lösung in Messküvetten und misst den Gehalt an Ru-Komplex fluoreszenzspektroskopisch. (Die Messbedingungen sind identisch mit jenen von Beispiel 12).

Die Resultate der r IFNαA Bestimmungen sind in Tabelle IV zusammengestellt.

Im Bereich von 0-200 U/ml findet man annähernd eine lineare Beziehung zwischen der Fluoreszenzintensität und der r IFNαA-Konzentration. Die Empfindlichkeit beträgt 0,46 U/ml r IFNαA.

Tabelle IV

Fluoreszenzspektroskopische Bestimmung von r IFNaA Standards

| Konzentration an r IFNαA (Roche Standard Lösungen) | Rel. Fluoreszenzintensität |
|---|---|
| 0 U/ml | 0,14 |
| 25 U/ml | 0,51 |
| 50 U/ml | 0,89 |
| 100 U/ml | 1,80 |
| 150 U/ml | 2,77 |
| 200 U/ml | 4,01 |

Beispiel 17

Markierung von polyklonalem anti-HCG mit dem Ru-Komnlex (RC-7]

Für die Kopplungsreaktion werden die folgenden Stammlösungen hergestellt:

1) 5,4 mg/ml Ru-Komplex [RC-7] in Wasser bei pH 4,5.

2) 6,0 mg/ml polyklonales anti-HCG vom Kaninchen (DAKO Code Nr. A 231, Lot 032A) in 200 mM $NaHCO_3$; pH 8,5.

Die Kopplungsreaktion wird nach dem im Beispiel 11 beschriebenen Verfahren durchgeführt. Man verwendet hierzu jeweils 400 ul von den Stammlösungen 1 und 2 sowie 0,56 mg vom wasserlöslichen Carbodiimid.

Die markierten Antikörper wurden - wie in Beispiel 11 beschrieben - durch Gelchromatographie vom Ueberschuss an Ru-Komplex [RC-7] abgetrennt.

Die UV-spektroskopische Bestimmung des Gehaltes an anti-HCG und an Ru-Komplex ergab folgende

Werte:
$3,81 \times 10^{-6}$ M/l Ru-Komplex
$1,87 \times 10^{-6}$ M/l anti-HCG (polyklonal).
Dies entspricht einem Markierungsgrad MG von 2,0.

Beispiel 18

Markierung von polyklonalem anti-CEA mit dem Ru-Komplex [RC-8]

Für die Kopplungsreaktion werden die folgenden Stammlösungen hergestellt:
1) 1,71 mg/ml Ru-Komplex [RC-8] in Wasser bei pH 4,5.
2) 2,17 mg/ml polyklonales anti-CEA vom Kaninchen (DAKO Code No. A115, Lot 112B) in 200 mM $NaHCO_3$; pH 8,5.

Die Kopplungsreaktion wird nach dem im Beispiel 11 beschriebenen Verfahren durchgeführt. Man verwendet hierzu jeweils 400 µl von den Stammlösungen 1 und 2 sowie 0,19 mg vom wasserlöslichen Carbodiimid.

Die markierten Antikörper wurden - wie in Beispiel 11 beschrieben - durch Gelchromatographie vom Ueberschuss an Ru-Komplex [RC-8] abgetrennt.

Die UV-spektroskopische Bestimmung des Gehaltes an anti-CEA und an Ru-Komplex ergab folgende Werte:
$0,43 \times 10^{-6}$ M/l Ru-Komplex
$0,94 \times 10^{-6}$ M/l anti-CEA
Dies entspricht einem Markierungsgrad MG von 0,5.

Beispiel 19

Markierung von h-IgG mit dem Ru-Komplex [RC-6]

Für die Kopplungsreaktion werden die folgenden Stammlösungen hergestellt:
1) 3,6 mg/ml Ru-Komplex [RC-6] in Wasser bei pH 4,5
2) 6,0 mg/ml h-IgG in 200 mM $NaHCO_3$; pH 8,5.

Die Kopplungsreaktion erfolgt nach dem in Beispiel 11 beschriebenen Verfahren. Dabei werden jeweils 400 µl von den Stammlösungen 1 und 2 sowie 0,37 mg vom wasserlöslichen Carbodiimid verwendet.

Die markierten Antikörper werden - wie in Beispiel 11 beschrieben - durch Gelchromatographie vom Ueberschuss an Ru-Komplex [RC-6] abgetrennt.

Die UV-spektroskopische Bestimmung des Gehaltes an h-IgG und an Ru-Komplex ergab folgende Werte:
$2,31 \times 10^{-6}$ M/l Ru-Komplex
$1,62 \times 10^{-6}$ M/l h-IgG
Dies entspricht einem Markierungsgrad MG von 1,4.

Beispiel 20

Markierung von h-IgG mit dem Ru-Komplex [RC-1]

Für die Kopplungsreaktion werden die folgenden Stammlösungen hergestellt:
1) 4,0 mg/ml Ru-Komplex [RC-1] in Wasser bei pH 4,5
2) 6,0 mg/ml h-IgG in 200 mM $NaHCO_3$; pH 8,5.

Die Kopplungsreaktion erfolgt nach dem in Beispiel 11 beschriebenen Verfahren. Dabei werden jeweils 400 µl von den Stammlösungen 1 und 2 sowie 1,11 mg vom wasserlöslichen Carbodiimid verwendet.

Die markierten Antikörper werden - wie in Beispiel 11 beschrieben - durch Gelchromatographie vom Ueberschuss an Ru-Komplex [RC-1] abgetrennt.

Die UV-spektroskopische Bestimmung des Gehaltes an h-IgG und an Ru-Komplex ergab folgende Werte:

$10{,}4 \times 10^{-6}$ M/l Ru-Komplex

$1{,}8 \times 10^{-6}$ M/l h-IgG

Dies entspricht einem Markierungsgrad MG von 5,8.

Beispiel 21

Markierung von h-IgG mit dem Ru-Komplex [RC-10]

Für die Kopplungsreaktion werden die folgenden Stammlösungen hergestellt:

1) 29,0 mg/ml Ru-Komplex [RC-10] in Wasser bei pH 4,5

2) 6,0 mg/ml h-IgG in 200 mM $NaHCO_3$; pH 8,5.

Die Kopplungsreaktion erfolgt nach dem in Beispiel 11 beschriebenen Verfahren. Dabei werden 526 µl von der Stammlösung 1 und 400 µl von der Stammlösung 2 sowie 7,45 mg vom wasserlöslichen Carbodiimid verwendet.

Die markierten Antikörper werden - wie in Beispiel 11 beschrieben - durch Gelchromatographie vom Ueberschuss an Ru-Komplex [RC-10] abgetrennt.

Die UV-spektroskopische Bestimmung des Gehaltes an h-IgG und an Ru-Komplex ergab folgende Werte:

$0{,}81 \times 10^{-6}$ M/1 Ru-Komplex

$0{,}78 \times 10^{-6}$ M/l h-IgG

Dies entspricht einem Markierungsgrad MG von 1,0.

Beispiel 22

Markierung von h-IgG mit dem Ru-Komplex [RC-3]

Für die Kopplungsreaktion werden die folgenden Stammlösungen hergestellt:

1) 3,7 mg/ml Ru-Komplex [RC-3] in Wasser bei pH 4,5

2) 6,0 mg/ml h-IgG in 200 mM $NaHCO_3$; pH 8,5.

Die Kopplungsreaktion erfolgt nach dem in Beispiel 11 beschriebenen Verfahren. Dabei werden jeweils 400 µl von den Stammlösungen 1 und 2 sowie 0,75 mg vom wasserlöslichen Carbodiimid verwendet.

Die markierten Antikörper werden - wie in Beispiel 11 beschrieben - durch Gelchromatographie vom Ueberschuss an Ru-Komplex [RC-3] abgetrennt.

Die UV-spektroskopische Bestimmung des Gehaltes an h-IgG und an Ru-Komplex ergab folgende Werte:

$4{,}41 \times 10^{-6}$ M/l Ru-Komplex

$1{,}21 \times 10^{-6}$ M/l h-IgG

Dies entspricht einem Markierungsgrad MG von 3,6.

Beispiel 23

Markierung von h-IgG mit dem Ru-Komplex [RC-4]

Für die Kopplungsreaktion werden die folgenden Stammlösungen hergestellt:

1) 3,81 mg/ml Ru-Komplex [RC-4] in Wasser bei pH 4,5

2) 6,0 mg/ml h-IgG in 200 mM $NaHCO_3$; pH 8,5.

Die Kopplungsreaktion erfolgt nach dem in Beispiel 11 beschriebenen Verfahren. Dabei werden jeweils 400 µl von den Stammlösungen 1 und 2 sowie 0,75 mg vom wasserlöslichen Carbodiimid verwendet.

Die markierten Antikörper werden - wie in Beispiel 11 beschrieben - durch Gelchromatographie vom Ueberschuss an Ru-Komplex [RC-4] abgetrennt.

Die UV-spektroskopische Bestimmung des Gehaltes an h-IgG und an Ru-Komplex ergab folgende Werte:

$5,51 \times 10^{-6}$M/l Ru-Komplex

$1,17 \times 10^{-6}$ M/l h-IgG

Dies entspricht einem Markierungsgrad MG von 4,70.

Beispiel 24

Markierung von h-IgG mit dem Ru-Komplex [RC-5]

Für die Kopplungsreaktion werden die folgenden Stammlösungen hergestellt:

1) 3,87 mg/ml Ru-Komplex [RC-5] in Wasser bei pH 4,5

2) 6,0 mg/ml h-IgG in 200 mM $NaHCO_3$; pH 8,5.

Die Kopplungsreaktion erfolgt nach dem in Beispiel 11 beschriebenen Verfahren. Dabei werden jeweils 400 µl von den Stammlösungen 1 und 2 sowie 0,75 mg vom wasserlöslichen Carbodiimid verwendet.

Die markierten Antikörper werden - wie in Beispiel 11 beschrieben - durch Gelchromatographie vom Ueberschuss an Ru-Komplex [RC-5] abgetrennt.

Die UV-spektroskopische Bestimmung des Gehaltes an h-IgG und an Ru-Komplex ergab folgende Werte:

$11,7 \times 10^{-6}$ M/l Ru-Komplex

$1,04 \times 10^{-6}$ M/l h-IgG

Dies entspricht einem Markierungsgrad MG von 11,2.

Beispiel 25

Markierung von h-IgG mit dem Ru-Komplex [RC-9]

Für die Kopplungsreaktion werden die folgenden Stammlösungen hergestellt:

1) 3,45 mg/ml Ru-Komplex [RC-9] in Wasser bei pH 4,5

2) 6,0 mg/ml h-IgG in 200 mM $NaHCO_3$; pH 8,5.

Die Kopplungsreaktion erfolgt nach dem in Beispiel 11 beschriebenen Verfahren. Dabei werden jeweils 400 µl von den Stammlösungen 1 und 2 sowie 0,375 µl vom wasserlöslichen Carbodiimid verwendet.

Die markierten Antikörper werden - wie in Beispiel 11 beschrieben - durch Gelchromatographie vom Ueberschuss an Ru-Komplex [RC-9] abgetrennt.

Die UV-spektroskopische Bestimmung des Gehaltes an h-IgG und an Ru-Komplex ergab folgende Werte:

$5,69 \times 10^{-6}$ M/l Ru-Komplex

$0,45 \times 10^{-6}$ M/l h-IgG

Dies entspricht einem Markierungsgrad MG von 12,8.

## Ansprüche

1. Rutheniumkomplexe, an die ein immunologisch aktives Material gekoppelt ist, wobei die Rutheniumkomplexe vor der Kupplung die allgemeine Formel

$$Ru^{2+}L_1L_2L_3 \qquad I$$

aufweisen,
worin $L_1$, $L_2$ und $L_3$ gleich oder verschieden sind und je einen bi- oder polycyclischen Liganden mit mindestens zwei stickstoffhaltigen Heterocyclen bedeuten, wobei mindestens einer dieser Liganden mit

mindestens einer wasserlöslich machenden Gruppe, wie $-SO_3H$ oder -COOH substituiert ist, und wobei mindestens einer dieser Liganden mit mindestens einer reaktiven gruppe, wie -COOH, direkt oder über eine Spacergruppe, substituiert ist, und wobei die Liganden $L_1$, $L_2$ und $L_3$ über Stickstoffatome an das Ruthenium gebunden sind.

**2.** Rutheniumkomplex nach Anspruch 1, dadurch gekennzeichnet, dass die Liganden $L_1$ bzw. $L_2$ 2,2'-Bipyridin-, 1,10-Phenanthrolin-, Bathophenanthrolin-oder Benzobathophenanthrolin-gruppen enthalten.

**3.** Rutheniumkomplex nach Anspruch 2, dadurch gekennzeichnet, dass die Liganden $L_1$ bzw. $L_2$ Bathophenanthrolin sind und mit Sulfonsäuregruppen als wasserlöslich machende Gruppen substituiert sind.

**4.** Rutheniumkomplex nach Anspruch 3, dadurch gekennzeichnet, dass die Sulfonsäuregruppen in Form der Salze vorliegen.

**5.** Rutheniumkomplex nach Anspruch 1, dadurch gekennzeichnet, dass der Ligand $L_3$ eine 2,2'-Bipyridin-, 1,10-Phenanthrolin-, Bathophenanthrolin- oder Benzobathophenanthrolin-gruppe enthält.

**6.** Rutheniumkomplex nach Anspruch 5, dadurch gekennzeichnet, dass der Ligand $L_3$ eine Bathophenanthrolin-oder Benzobathophenanthrolin-gruppe enthält, die mit mindestens einer Alkylengruppe mit höchstens 8 C-Atomen substituiert ist, die $-SO_2$-NH- oder -COO- aufweisen kann.

**7.** Rutheniumkomplex nach Anspruch 6, dadurch gekennzeichnet, dass die Alkylengruppe endständig mit einer -COOH oder $-SO_2Hal$ Gruppe substituiert ist.

**8.** Rutheniumkomplex nach einem der Ansprüche 6-7, dadurch gekennzeichnet, dass der Ligand $L_3$ die Gruppe $-SO_2-NH(CH_2)_nCOOH$ enthält, wobei n eine ganze Zahl von 1-5 bedeutet.

**9.** Rutheniumkomplex nach einem der Ansprüche 6-7, dadurch gekennzeichnet, dass der Ligand $L_3$ die Gruppe $-(CH_2)_4COOH$ enthält.

**10.** Rutheniumkomplex nach einem der Ansprüche 6-7, dadurch gekennzeichnet, dass der Ligand $L_3$ die Gruppe $-(CH_2)_5COOH$ enthält.

**11.** Rutheniumkomplex nach Anspruch 5, dadurch gekennzeichnet, dass der Ligand $L_3$ 2,2'-Bipyridin ist, der mit mindestens einer Carboxylgruppe substituiert ist.

**12.** Rutheniumkomplex nach Anspruch 1, dadurch gekennzeichnet, dass die Liganden $L_1$, $L_2$ und $L_3$ gleich sind und Bathophenanthrolin darstellen, die sowohl mit einer wasserlöslich machenden Gruppe, wie $-SO_3H$ oder -COOH, als auch über eine Spacergruppe mit einer reaktiven Gruppe, wie -COOH, substituiert sind.

**13.** Rutheniumkomplexe nach Anspruch 12, dadurch gekennzeichnet, dass die wasserlöslich machende Gruppe eine Sulfonsäuregruppe ist und dass die reaktive Gruppe eine Carboxylgruppe ist, die über eine $-SO_2-NH-(CH_2)_2$-Spacergruppe verknüpft ist.

**14.** Rutheniumkomplex nach einem der Ansprüche 1-13, dadurch gekennzeichnet, dass das immunologisch aktive Material ein Antigen oder Hapten ist.

**15.** Rutheniumkomplex nach einem der Ansprüche 1-13, dadurch gekennzeichnet, dass das immunologisch aktive Material ein Antikörper ist.

**16.** Rutheniumkomplex nach Anspruch 15, dadurch gekennzeichnet, dass das immunologisch aktive Material ein Antikörper gegen carcinoembryonales Antigen, humanes Choriongonadotropin oder $\alpha$-Interferon ist.

**17.** Verfahren zur Herstellung eines Rutheniumkomplexes gemäss allgemeinen Formel I von Patentanspruch 1, an den ein immunologisch aktives Material gekoppelt ist, dadurch gekennzeichnet, dass man den Rutheniumkomplex gemäss allgemeiner Formel I in an sich bekannter Weise mit einem immunologisch aktiven Material koppelt.

18. Verfahren nach Anspruch 17, dadurch gekennzeichnet, dass man als Kopplungsmittel N-Cyclohexyl-N'-(2-morpholinoäthyl)-carbodiimid -methyl-p-toluolsulfonamid verwendet.

19. Verfahren nach Anspruch 17 oder 18, dadurch gekennzeichnet, dass man als immunologisches Material Antikörper gegen carcinoembryonales Antigen, humanes Choriongonadotropin oder α-Interferon verwendet.

20. Verwendung eines Rutheniumkomplexes nach einem der Ansprüche 1-13 in einem Fluoreszenzimmunoassay.

21. Verwendung eines Rutheniumkomplexes nach einem der Ansprüche 1-13 in einem Fluoreszenzimmunoassay mit zeitaufgelöster Fluoreszenzmessung.

22. Rutheniumkomplexe der allgemeinen Formel

$$Ru^{2+}L_1L_2L_3 \qquad I$$

worin $L_1$, $L_2$ und $L_3$ gleich oder verschieden sind und je einen bi- oder polycyclischen Liganden mit mindestens zwei stickstoffhaltigen Heterocyclen bedeuten, wobei mindestens einer dieser Liganden mit mindestens einer wasserlöslich machenden Gruppe wie $-SO_3H$ oder -COOH substituiert ist, und wobei mindestens einer dieser Ligande mit mindestens einer reaktiven Gruppe, wie -COOH direkt oder über einer Spacergruppe, substituiert ist, und wobei die Liganden $L_1$, $L_2$ und $L_3$ über Stickstoffatome an das Ruthenium gebunden sind.

23. Rutheniumkomplexe gemäss Anspruch 22, worin die Liganden $L_1$, $L_2$ und $L_3$ die in den Ansprüchen 2-13 erwähnten Bedeutungen haben.

Patentansprüche für folgenden Vertragsstaat: AT

1. Verfahren zur Herstellung eines Rutheniumkomplexes gemäss allgemeinen Formel

$$Ru^{2+}L_1L_2L_3 \qquad I$$

worin $L_1$, $L_2$ und $L_3$ gleich oder verschieden sind und je einen bi- oder polycyclischen Liganden mit mindestens zwei stickstoffhaltigen Heterocyclen bedeuten, wobei mindestens einer dieser Liganden mit mindestens einer wasserlöslich machenden Gruppe, wie $-SO_3H$ oder -COOH substituiert ist, und wobei mindestens einer dieser Liganden mit mindstens einer reaktiven Gruppe, wie -COOH direkt oder über eine Spacergruppe, substituiert ist, und wobei die Liganden $L_1$, $L_2$ und $L_3$ über Stickstoffatome an das Ruthenium gebunden sind,
an den ein immunologisch aktives Material gekoppelt ist, dadurch gekennzeichnet, dass man den Rutheniumkomplex gemäss allgemeiner Formel I mittels eines chemischen Kopplungmittels an ein immunologisch aktives Material koppelt.

2. Verfahren nach Anspruch 1, dadurch gekennzeichnet, dass man als Kopplungsmittel N-Cyclohexyl-N'-(2-morpholinoäthyl)-carbodiimid -methyl-p-toluolsulfonamid verwendet.

3. Verfahren nach Anspruch 1 oder 2, dadurch gekennzeichnet, dass man als immunologisches Material Antikörper gegen carcinoembryonales Antigen, humanes Choriongonadotropin oder α-Interferon verwendet.

**Claims**

1. Ruthenium complexes to which is coupled an immunologically active material, whereby the ruthenium complexes, before the coupling, have the general formula

$$Ru^{2+}L_1L_2L_3 \qquad I$$

wherein $L_1$. $L_2$ and $L_3$ are the same or different and each signify a bi- or polycyclic ligand with at least two nitrogen-containing heterocycles, whereby at least one of these ligands is substituted with at least one group, such as $-SO_3H$ or -COOH, conferring water-solubility, and whereby at least one of these ligands is substituted, directly or via a spacer group, with at least one reactive group, such as -COOH, and whereby the ligands $L_1$. $L_2$ and $L_3$ are attached to the ruthenium via nitrogen atoms.

2. A ruthenium complex according to claim 1, characterized in that the ligands $L_1$ and $L_2$ contain 2,2'-bipyridine, 1,10-phenanthroline, bathophenanthroline or benzobathophenanthroline groups.

3. A ruthenium complex according to claim 2. characterized in that the ligands $L_1$ and $L_2$ are bathophenanthroline and are substituted with sulphonic acid groups as groups conferring water-solubility.

4. A ruthenium complex according to claim 3, characterized in that the sulphonic acid groups are present in the form of salts.

5. A ruthenium complex according to claim 1, characterized in that the ligand $L_3$ contains a 2,2'-bipyridine, 1.10-phenanthroline, bathophenanthroline or benzobathophenanthroline group.

6. A ruthenium complex according to claim 5, characterized in that the ligand $L_3$ contains a bathophenanthroline or benzobathophenanthroline group which is substituted with at least one alkylene group having a maximum of 8 C atoms and which can contain $-SO_2$-NH- or -COO-.

7. A ruthenium complex according to claim 6, characterized in that the alkylene group is substituted terminally with a -COOH or $-SO_2Hal$ group.

8. A ruthenium complex according to one of claims 6-7, characterized in that the ligand $L_3$ contains the group $-SO_2-NH(CH_2)_nCOOH$ in which n signifies a whole number of 1-5.

9. A ruthenium complex according to one of claims 6-7, characterized in that the ligand $L_3$ contains the group $-(CH_2)_4COOH$.

10. A ruthenium complex according to one of claims 6-7, characterized in that the ligand $L_3$ contains the group $-(CH_2)_5COOH$.

11. A ruthenium complex according to claim 5, characterized in that the ligand $L_3$ is 2,2'-bipyridine which is substituted with at least one carboxyl group.

12. A ruthenium complex according to claim 1, characterized in that the ligands $L_1$, $L_2$ and $L_3$ are the same and represent bathophenanthroline which are substituted not only with a group, such as $-SO_3H$ or -COOH, conferring water-solubility, but also via a spacer group with a reactive group, such as -COOH.

13. Ruthenium complexes according to claim 12, characterized in that the group conferring water-solubility is a sulphonic acid group and in that the reactive group is a carboxyl group which is linked via a $-SO_2-NH-(CH_2)_2-$ spacer group.

14. A ruthenium complex according to any one of claims 1-13, characterized in that the immunologically active material is an antigen or hapten.

15. A ruthenium complex according to any one of claims 1-13, characterized in that the immunologically active material is an antibody.

**16.** A ruthenium complex according to claim 15, characterized in that the immunologically active material is an antibody against carcinoembryonal antigen, human choriongonadotropin or α-interferon.

**17.** A process for the manufacture of a ruthenium complex in accordance with general formula I of Patent claim 1 to which is coupled an immunologically active material, characterized by coupling the ruthenium complex in accordance with general formula I with an immunologically active material in a manner known per se.

**18.** A process according to claim 17, characterized in that N-cyclohexyl-N'-(2-morpholinoethyl)-carbodiimide--methyl-p-toluenesulphonamide is used as the coupling agent.

**19.** A process according to claim 17 or 18, characterized in that an antibody against carcinoembryonal antigen, human choriongonadotropin or α-interferon is used as the immunological material.

**20.** The use of a ruthenium complex according to any one of claims 1-13 in a fluorescence immunoassay.

**21.** The use of a ruthenium complex according to any one of claims 1-13 in a fluorescence immunoassay with time-resolved fluorescence measurement.

**22.** Ruthenium complexes of the general formula

$$Ru^{2+}L_1L_2L_3 \qquad I$$

wherein $L_1$, $L_2$ and $L_3$ are the same or different and each signify a bi- or polycyclic ligand with at least two nitrogen-containing heterocycles, whereby at least one of these ligands is substituted with at least one group, such as $-SO_3H$ or -COOH, conferring water-solubility, and whereby at least one of these ligands is substituted, directly or via a spacer group, with at least one reactive group, such as -COOH, and whereby the ligands $L_1$, $L_2$ and $L_3$ are attached to the ruthenium via nitrogen atoms.

**23.** Ruthenium complexes in accordance with claim 22, wherein the ligands $L_1$, $L_2$ and $L_3$ have the significances set forth in claims 2-13.

Claims for the following Contracting State: AT

**1.** A process for the manufacture of a ruthenium complex in accordance with the general formula

$$Ru^{2+}L_1L_2L_3 \qquad I$$

wherein $L_1$, $L_2$ and $L_3$ are the same or different and each signify a bi- or polycyclic ligand with at least two nitrogen-containing heterocycles, whereby at least one of these ligands is substituted with at least one group, such as $-SO_3H$ or -COOH, conferring water-solubility, and whereby at least one of these ligands is substituted, directly or via a spacer group, with at least one reactive group, such as -COOH, and whereby the ligands $L_1$, $L_2$ and $L_3$ are attached to the ruthenium via nitrogen atoms,
to which is coupled an immunologically active material, characterized by coupling the ruthenium complex in accordance with general formula I to an immunologically active material using a chemical coupling agent.

**2.** A process according to claim 1, characterized in that N-cyclohexyl-N'-(2-morpholinoethyl)-carbodiimide--methyl-p-toluenesulphonamide is used as the coupling agent.

**3.** A process according to claim 1 or 2, characterized in that an antibody against carcinoembryonal antigen, human choriongonadotropin or α-interferon is used as the immunological material.

**Revendications**

1. Complexes du ruthénium, sur lesquels on a couplé une matière possédant une activité immunologique, les complexes de ruthénium répondant, avant le couplage, à la formule générale :

$$Ru^{2+}L_1L_2L_3 \qquad I$$

dans laquelle $L_1$, $L_2$ et $L_3$, ayant des significations identiques ou différentes, représentent chacun un ligand bi- ou polycyclique contenant au moins deux hétérocycles azotés, l'un au moins de ces ligands étant substitué par au moins un groupe hydrosolubilisant tel que $-SO_3H$ ou -COOH, l'un au moins de ces ligands étant substitué directement ou par l'intermédiaire d'un pont par au moins un groupe réactif tel que -COOH, et les ligands $L_1$, $L_2$ et $L_3$ sont reliés au ruthénium par l'intermédiaire d'atomes d'azote.

2. Complexe de ruthénium selon la revendication 1, caractérisé en ce que les ligands $L_1$ et $L_2$ contiennent respectivement des groupes 2,2'-bipyridine, 1,10-phénanthroline, bathophénanthroline ou benzobathophénanthroline.

3. Complexe de ruthénium selon la revendication 2, caractérisé en ce que les ligands $L_1$ et/ou $L_2$ consistent en bathophénanthroline et sont substitués par des groupes acides sulfoniques représentant les groupes hydrosolubilisants.

4. Complexe de ruthénium selon la revendication 3, caractérisé en ce que les groupes acides sulfoniques sont à l'état de sels.

5. Complexe de ruthénium selon la revendication 1, caractérisé en ce que le ligand $L_3$ contient un groupe 2,2'-bipyridine, 1,10-phénanthroline, bathophénanthroline ou benzobathophénanthroline.

6. Complexe de ruthénium selon la revendication 5, caractérisé en ce que le ligand $L_3$ contient un groupe bathophénanthroline ou benzobathophénanthroline substitué par au moins un groupe alkylène à 8 atomes de carbone au maximum, qui peut contenir un groupe $-SO_2$-NH- ou -COO-.

7. Complexe de ruthénium selon la revendication 6, caractérisé en ce que le groupe alkylène est substitué en position terminale par un groupe -COOH ou $-SO_2Hal$.

8. Complexe de ruthénium selon l'une des revendications 6 ou 7, caractérisé en ce que le ligand $L_3$ contient le groupe $-SO_2-NH(CH_2)_nCOOH$ dans lequel n est un nombre entier allant de 1 à 5.

9. Complexe de ruthénium selon l'une des revendications 6 ou 7, caractérisé en ce que le ligand $L_3$ contient le groupe $-(CH_2)_4COOH$.

10. Complexe de ruthénium selon l'une des revendications 6 ou 7, caractérisé en ce que le ligand $L_3$ contient le groupe $-(CH_2)_5COOH$.

11. Complexe de ruthénium selon la revendication 5, caractérisé en ce que le ligand $L_3$ est une 2,2'-bipyridine substituée par au moins un groupe carboxyle.

12. Complexe de ruthénium selon la revendication 1, caractérisé en ce que les ligands $L_1$, $L_2$ et $L_3$ sont identiques et consistent en une bathophénanthroline qui est substituée à la fois par un groupe hydrosolubilisant tel que $-SO_3H$ ou -COOH et, par l'intermédiaire d'un pont, par un groupe réactif tel que -COOH.

13. Complexes de ruthénium selon la revendication 12, caractérisés en ce que le groupe hydrosolubilisant est un groupe acide sulfonique et en ce que le groupe réactif est un groupe carboxyle relié par l'intermédiaire d'un pont $-SO_2-NH-(CH_2)_2-$.

14. Complexe de ruthénium selon l'une des revendications 1 à 13, caractérisé en ce que la matière possédant une activité immunologique est un antigène ou un haptène.

**15.** Complexe de ruthénium selon l'une des revendications 1 à 13, caractérisé en ce que la matière possédant l'activité immunologique est un anticorps.

**16.** Complexe de ruthénium selon la revendication 15, caractérisé en ce que la matière possédant l'activité immunologique est un anticorps contre un antigène carcinoembryonnaire, une chorionogonadotropine humaine ou l'alpha-interféron.

**17.** Procédé de préparation d'un complexe de ruthénium de formule générale I de la revendication 1, sur lequel on a couplé une matière possédant une activité immunologique, caractérisé en ce que l'on couple le complexe de ruthénium de formule générale I, de manière connue en soi, avec une matière possédant l'activité immunologique.

**18.** Procédé selon la revendication 17, caractérisé en ce que l'on utilise en tant qu'agent couplant le N-cyclohexyl-N'-(2-morpholinoéthyl)-carbodiimido-méthyl-p-toluènesulfonamide.

**19.** Procédé selon la revendication 17 ou 18, caractérisé en ce que l'on utilise en tant que matière immunologique un anticorps contre un antigène carcinoembryonnaire, la chorionogonadotropine humaine ou l'alpha-interféron.

**20.** Utilisation d'un complexe de ruthénium selon l'une des revendications 1 à 13, dans un immunotest par fluorescence.

**21.** Utilisation d'un complexe de ruthénium selon l'une des revendications 1 à 13, dans un immunotest par fluorescence avec mesure de la fluorescence dispersée dans le temps.

**22.** Complexes de ruthénium de formule générale :

$$Ru^{2+}L_1L_2L_3$$

dans laquelle $L_1$, $L_2$ et $L_3$, ayant des significations identiques ou différentes, représentent chacun un ligand bi- ou polycyclique à au moins deux hétérocycles azotés, l'un au moins de ces ligands étant substitué par au moins un groupe hydrosolubilisant tel que $-SO_3H$ ou -COOH, l'un au moins de ces ligands étant substitué, directement ou par l'intermédiaire d'un pont, par au moins un groupe réactif tel que -COOH, et les ligards $L_1$, $L_2$ et $L_3$ étant reliés au ruthénium par l'intermédiaire d'atomes d'azote.

**23.** Complexes de ruthénium selon la revendication 22, dans lesquels les ligands $L_1$ , $L_2$ et $L_3$ ont les significations indiquées dans les revendications 2 à 13.

Revendications pour l'Etats contractant suivant: AT

**1.** Procédé de préparation d'un complexe de ruthénium de formule générale :

$$Ru^{2+}L_1L_2L_3$$

dans laquelle $L_1$, $L_2$ et $L_3$, ayant des significations identiques ou différentes, représentent chacun un ligand bi- ou polycyclique à au moins deux hétérocycles azotés, l'un au moins de ces ligands étant substitué par au moins un groupe hydrosolubilisant tel que $-SO_3H$ ou -COOH, l'un au moins de ces ligands étant substitué, directement ou par l'intermédiaire d'un pont, par au moins un groupe réactif tel que -COOH, et les ligands $L_1$, $L_2$ et $L_3$ étant reliés au ruthénium par l'intermédiaire d'atomes d'azote,
sur lequel on a couplé une matière possédant une activité immunologique, caractérisé en ce que l'on couple le complexe de ruthénium de formule générale I, à l'aide d'un agent couplant chimique, sur une matière possédant une activité immunologique.

**2.** Procédé selon la revendication 1, caractérisé en ce que l'on utilise en tant qu'agent couplant le N-cyclohexyl-N'-(2-morpholinoéthyl)-carbodiimidométhyl-p-toluène-sulfonamide.

3. Procédé selon la revendication 1 ou 2, caractérisé en ce que l'on utilise en tant que matière immunologique un anticorps contre l'antigène carcinoembryonnaire, la chorionogonadotropine humaine ou l'alpha-interféron.